(19)

Europäisches
Patentamt
European
Patent Office
Office européen
des brevets

(11) **EP 2 619 231 B1**

(12) **EUROPEAN PATENT SPECIFICATION**

(45) Date of publication and mention
of the grant of the patent:
**21.12.2016   Bulletin 2016/51**

(21) Application number: **11827625.2**

(22) Date of filing: **23.09.2011**

(51) Int Cl.:
*C08B 37/08* [(2006.01)]     *G01N 33/50* [(2006.01)]

(86) International application number:
**PCT/US2011/053015**

(87) International publication number:
**WO 2012/040592 (29.03.2012 Gazette 2012/13)**

(54) **METHODS FOR THE EVALUATION OF RENAL INJURY USING HYALURONIC ACID**

VERFAHREN ZUR BEWERTUNG VON NIERENLÄSIONEN MITHILFE VON HYALURONSÄURE

PROCÉDÉS DESTINÉS À L'ÉVALUATION D'UNE LÉSION RÉNALE AU MOYEN D'ACIDE
HYALURONIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **24.09.2010   US 386421 P**

(43) Date of publication of application:
**31.07.2013   Bulletin 2013/31**

(73) Proprietors:
• **Astute Medical, Inc.
San Diego, CA 92121 (US)**
• **University of Pittsburgh - Of the Commonwealth
System of Higher Education
Pittsburgh, PA 15260 (US)**

(72) Inventors:
• **ANDERBERG, Joseph
Encinitas
CA 92024 (US)**
• **GRAY, Jeff
Solana Beach
CA92075 (US)**
• **MCPHERSON, Paul
Encinitas
CA 92024 (US)**
• **NAKAMURA, Kevin
Cardiff by the Sea
CA 92007 (US)**
• **KAMPF, James, Patrick
San Diego
CA 92130 (US)**
• **SINGBARTL, Kai
Pittsburgh, Pennsylvannia 15206 (US)**

• **KELLUM, John A. Jr.
Pittsburgh, Pennsylvannia 15206 (US)**

(74) Representative: **Schiweck, Weinzierl & Koch
European Patent Attorneys
Landsberger Straße 98
80339 München (DE)**

(56) References cited:
**WO-A1-2010/091231     US-A1- 2009 298 106
US-A1- 2010 081 148     US-A1- 2010 190 164**

• **ROBERTO PECOITS-FILHO ET AL:
"Associations between circulating inflammatory
markers and residual renal function in CRF
patients", AMERICAN JOURNAL OF KIDNEY
DISEASES, vol. 41, no. 6, 1 June 2003
(2003-06-01), pages 1212-1218, XP055045573,
ISSN: 0272-6386, DOI:
10.1016/S0272-6386(03)00353-6**
• **RICHARD Y LIN ET AL: "Urinary hyaluronic acid
is a Wilms' tumor marker", JOURNAL OF
PEDIATRIC SURGERY, vol. 30, no. 2, 1 February
1995 (1995-02-01), pages 304-308, XP055107750,
ISSN: 0022-3468, DOI:
10.1016/0022-3468(95)90578-2**
• **MARINO ASSELMAN ET AL: "Hyaluronan and
Stone Disease", AIP CONFERENCE
PROCEEDINGS, vol. 1049, 1 January 2008
(2008-01-01), pages 133-144, XP055108502, ISSN:
0094-243X, DOI: 10.1063/1.2998010**

Note: Within nine months of the publication of the mention of the grant of the European patent in the European Patent
Bulletin, any person may give notice to the European Patent Office of opposition to that patent, in accordance with the
Implementing Regulations. Notice of opposition shall not be deemed to have been filed until the opposition fee has been
paid. (Art. 99(1) European Patent Convention).

**(Cont. next page)**

EP 2 619 231 B1

EP 2 619 231 B1

- SHEN WENQING ET AL: "Changes and clinical significance of hyaluronic acid , procollagen type III, collagen type IV and laminin in the serum and urine in patients with chronic renal diseases", LINCHUANG YIXUE (ZHENGZHOU), CN, vol. 30, no. 6, 1 June 2010 (2010-06-01), pages 4-7, XP009177055, ISSN: 1003-3548
- ZHANG ET AL: "Clinical significance of determination of changes of serum hyaluronic acid (HA) and plasma vascular endothelial growth factor (VEGF) contents after treatment in patients with chronic nephropathy", FANGSHE MIANYIXUE ZAZHI,, vol. 19, no. 4, 1 January 2006 (2006-01-01), pages 286-287, XP009177083,
- HONKANEN E ET AL: "Serum hyaluronic acid and procollagen III amino terminal propeptide in chronic renal failure", AMERICAN JOURNAL OF NEPHROLOGY, S. KARGER AG, CH, vol. 11, no. 3, 1 January 1991 (1991-01-01), pages 201-206, XP009177043, ISSN: 0250-8095
- HAELLGREN R ET AL: "Circulating hyaluronate. A potential marker of altered metabolism of the connective tissue in uremia", NEPHRON EXPERIMENTAL NEPHROLOGY, S. KARGER AG, SWITZERLAND, vol. 46, no. 2, 1 January 1987 (1987-01-01), pages 150-154, XP009177041, ISSN: 0028-2766
- RICCI ET AL.: 'The RIFLE criteria and mortality in acute kidney injury: A systematic review' KIDNEY INTERNATIONAL vol. 73, 2008, pages 538 - 546, XP055106362

**Description**

The present application claims priority to U.S. Provisional Patent Application 61/386,421 filed September 24, 2010.

This invention was made with U.S. government support under Grant/Contract No. 5R01DK070910-035R01DK070910-03 awarded by the National Institutes of Diabetes and Digestive and Kidney Diseases. The government has certain rights in the invention.

BACKGROUND OF THE INVENTION

The following discussion of the background of the invention is merely provided to aid the reader in understanding the invention and is not admitted to describe or constitute prior art to the present invention.

The kidney is responsible for water and solute excretion from the body. Its functions include maintenance of acid-base balance, regulation of electrolyte concentrations, control of blood volume, and regulation of blood pressure. As such, loss of kidney function through injury and/or disease results in substantial morbidity and mortality. A detailed discussion of renal injuries is provided in Harrison's Principles of Internal Medicine, 17th Ed., McGraw Hill, New York, pages 1741-1830. Renal disease and/or injury may be acute or chronic. Acute and chronic kidney disease are described as follows (from Current Medical Diagnosis & Treatment 2008, 47th Ed, McGraw Hill, New York, pages 785-815): "Acute renal failure is worsening of renal function over hours to days, resulting in the retention of nitrogenous wastes (such as urea nitrogen) and creatinine in the blood. Retention of these substances is called azotemia. Chronic renal failure (chronic kidney disease) results from an abnormal loss of renal function over months to years".

Acute renal failure (ARF, also known as acute kidney injury, or AKI) is an abrupt (typically detected within about 48 hours to 1 week) reduction in glomerular filtration. This loss of filtration capacity results in retention of nitrogenous (urea and creatinine) and non-nitrogenous waste products that are normally excreted by the kidney, a reduction in urine output, or both. It is reported that ARF complicates about 5% of hospital admissions, 4-15% of cardiopulmonary bypass surgeries, and up to 30% of intensive care admissions. ARF may be categorized as prerenal, intrinsic renal, or postrenal in causation. Intrinsic renal disease can be further divided into glomerular, tubular, interstitial, and vascular abnormalities. Major causes of ARF are described in the following table, which is adapted from the Merck Manual, 17th ed., Chapter 222:

| Type | Risk Factors |
|---|---|
| **Prerenal** | |
| ECF volume depletion | Excessive diuresis, hemorrhage, GI losses, loss of intravascular fluid into the extravascular space (due to ascites, peritonitis, pancreatitis, or burns), loss of skin and mucus membranes, renal salt- and water-wasting states |
| Low cardiac output | Cardiomyopathy, MI, cardiac tamponade, pulmonary embolism, pulmonary hypertension, positive-pressure mechanical ventilation |
| Low systemic vascular resistance | Septic shock, liver failure, antihypertensive drugs |
| Increased renal vascular resistance | NSAIDs, cyclosporines, tacrolimus, hypercalcemia, anaphylaxis, anesthetics, renal artery obstruction, renal vein thrombosis, sepsis, hepatorenal syndrome |
| Decreased efferent arteriolar tone (leading to decreased GFR from reduced glomerular transcapillary pressure, especially in patients with bilateral renal artery stenosis) | ACE inhibitors or angiotensin II receptor blockers |
| **Intrinsic Renal** | |
| Acute tubular injury | Ischemia (prolonged or severe prerenal state): surgery, hemorrhage, arterial or venous obstruction; Toxins: NSAIDs, cyclosporines, tacrolimus, aminoglycosides, foscarnet, ethylene glycol, hemoglobin, myoglobin, ifosfamide, heavy metals, methotrexate, radiopaque contrast agents, streptozotocin |

(continued)

| Intrinsic Renal | |
| --- | --- |
| Acute glomerulonephritis | ANCA-associated: Crescentic glomerulonephritis, polyarteritis nodosa, Wegener's granulomatosis; Anti-GBM glomerulonephritis: Goodpasture's syndrome; Immune-complex: Lupus glomerulonephritis, postinfectious glomerulonephritis, cryoglobulinemic glomerulonephritis |
| Acute tubulo interstitial nephritis | Drug reaction (eg, $\beta$-lactams, NSAIDs, sulfonamides, ciprofloxacin, thiazide diuretics, furosemide, phenytoin, allopurinol, pyelonephritis, papillary necrosis |
| Acute vascular nephropathy | Vasculitis, malignant hypertension, thrombotic microangiopathies, scleroderma, atheroembolism |
| Infiltrative diseases | Lymphoma, sarcoidosis, leukemia |
| **Postrenal** | |
| Tubular precipitation | Uric acid (tumor lysis), sulfonamides, triamterene, acyclovir, indinavir, methotrexate, ethylene glycol ingestion, myeloma protein, myoglobin |
| **Type** | **Risk Factors** |
| Ureteral obstruction | Intrinsic: Calculi, clots, sloughed renal tissue, fungus ball, edema, malignancy, congenital defects; Extrinsic: Malignancy, retroperitoneal fibrosis, ureteral trauma during surgery or high impact injury |
| Bladder obstruction | Mechanical: Benign prostatic hyperplasia, prostate cancer, bladder cancer, urethral strictures, phimosis, paraphimosis, urethral valves, obstructed indwelling urinary catheter; Neurogenic: Anticholinergic drugs, upper or lower motor neuron lesion |

In the case of ischemic ARF, the course of the disease may be divided into four phases. During an initiation phase, which lasts hours to days, reduced perfusion of the kidney is evolving into injury. Glomerular ultrafiltration reduces, the flow of filtrate is reduced due to debris within the tubules, and back leakage of filtrate through injured epithelium occurs. Renal injury can be mediated during this phase by reperfusion of the kidney. Initiation is followed by an extension phase which is characterized by continued ischemic injury and inflammation and may involve endothelial damage and vascular congestion. During the maintenance phase, lasting from 1 to 2 weeks, renal cell injury occurs, and glomerular filtration and urine output reaches a minimum. A recovery phase can follow in which the renal epithelium is repaired and GFR gradually recovers. Despite this, the survival rate of subjects with ARF may be as low as about 60%.

Acute kidney injury caused by radiocontrast agents (also called contrast media) and other nephrotoxins such as cyclosporine, antibiotics including aminoglycosides and anticancer drugs such as cisplatin manifests over a period of days to about a week. Contrast induced nephropathy (CIN, which is AKI caused by radiocontrast agents) is thought to be caused by intrarenal vasoconstriction (leading to ischemic injury) and from the generation of reactive oxygen species that are directly toxic to renal tubular epithelial cells. CIN classically presents as an acute (onset within 24-48h) but reversible (peak 3-5 days, resolution within 1 week) rise in blood urea nitrogen and serum creatinine.

A commonly reported criteria for defining and detecting AKI is an abrupt (typically within about 2-7 days or within a period of hospitalization) elevation of serum creatinine. Although the use of serum creatinine elevation to define and detect AKI is well established, the magnitude of the serum creatinine elevation and the time over which it is measured to define AKI varies considerably among publications. Traditionally, relatively large increases in serum creatinine such as 100%, 200%, an increase of at least 100% to a value over 2 mg/dL and other definitions were used to define AKI. However, the recent trend has been towards using smaller serum creatinine rises to define AKI. The relationship between serum creatinine rise, AKI and the associated health risks are reviewed in Praught and Shlipak, Curr Opin Nephrol Hypertens 14:265-270, 2005 and Chertow et al, J Am Soc Nephrol 16: 3365-3370, 2005. As described in these publications, acute worsening renal function (AKI) and increased risk of death and other detrimental outcomes are now known to be associated with very small increases in serum creatinine. These increases may be determined as a relative (percent) value or a nominal value. Relative increases in serum creatinine as small as 20% from the pre-injury value have been reported to indicate acutely worsening renal function (AKI) and increased health risk, but the more commonly reported value to define AKI and increased health risk is a relative increase of at least 25%. Nominal increases as small as 0.3 mg/dL, 0.2 mg/dL or even 0.1 mg/dL have been reported to indicate worsening renal function and increased risk of death. Various time periods for the serum creatinine to rise to these threshold values have been used to define AKI, for example, ranging from 2 days, 3 days, 7 days, or a variable period defined as the time the patient is in the hospital or intensive care unit. These studies indicate there is not a particular threshold serum creatinine rise (or time period for the rise) for worsening renal function or AKI, but rather a continuous increase in risk with increasing magnitude of serum creatinine rise.

One study (Lassnigg et all, J Am Soc Nephrol 15:1597-1605, 2004) investigated both increases and decreases in serum creatinine. Patients with a mild fall in serum creatinine of -0.1 to -0.3 mg/dL following heart surgery had the lowest mortality rate. Patients with a larger fall in serum creatinine (more than or equal to -0.4 mg/dL) or any increase in serum creatinine had a larger mortality rate. These findings caused the authors to conclude that even very subtle changes in renal function (as detected by small creatinine changes within 48 hours of surgery) seriously effect patient's outcomes. In an effort to reach consensus on a unified classification system for using serum creatinine to define AKI in clinical trials and in clinical practice, Bellomo et al., Crit Care. 8(4):R204-12, 2004 proposes the following classifications for stratifying AKI patients:

"Risk": serum creatinine increased 1.5 fold from baseline OR urine production of <0.5 ml/kg body weight/hr for 6 hours;

"Injury": serum creatinine increased 2.0 fold from baseline OR urine production <0.5 ml/kg/hr for 12 h;

"Failure": serum creatinine increased 3.0 fold from baseline OR creatinine >355 $\mu$mol/l (with a rise of >44) or urine output below 0.3 ml/kg/hr for 24 h or anuria for at least 12 hours;

And included two clinical outcomes:

"Loss": persistent need for renal replacement therapy for more than four weeks.

"ESRD": end stage renal disease-the need for dialysis for more than 3 months.

These criteria are called the RIFLE criteria, which provide a useful clinical tool to classify renal status. As discussed in Kellum, Crit. Care Med. 36: S 141-45, 2008 and Ricci et al., Kidney Int. 73, 538-546, 2008 the RIFLE criteria provide a uniform definition of AKI which has been validated in numerous studies. For purposes of the present invention, "RIFLE stage 0" refers to a patient that does not fall within the RIFLE R, I or F criteria, and so is "pre-risk."

More recently, Mehta et al., Crit. Care 11:R31 (doi:10.1186.cc5713), 2007 proposes the following similar classifications for stratifying AKI patients, which have been modified from RIFLE:

"Stage I": increase in serum creatinine of more than or equal to 0.3 mg/dL ($\geq$ 26.4 $\mu$mol/L) or increase to more than or equal to 150% (1.5-fold) from baseline OR urine output less than 0.5 mL/kg per hour for more than 6 hours;

"Stage II": increase in serum creatinine to more than 200% (> 2-fold) from baseline OR urine output less than 0.5 mL/kg per hour for more than 12 hours;

"Stage III": increase in serum creatinine to more than 300% (> 3-fold) from baseline OR serum creatinine $\geq$ 354 $\mu$mol/L accompanied by an acute increase of at least 44 $\mu$mol/L OR urine output less than 0.3 mL/kg per hour for 24 hours or anuria for 12 hours.

The CIN Consensus Working Panel (McCollough et al, Rev Cardiovasc Med. 2006;7(4):177-197) uses a serum creatinine rise of 25% to define Contrast induced nephropathy (which is a type of AKI).Although various groups propose slightly different criteria for using serum creatinine to detect AKI, the consensus is that small changes in serum creatinine, such as 0.3 mg/dL or 25%, are sufficient to detect AKI (worsening renal function) and that the magnitude of the serum creatinine change is an indicator of the severity of the AKI and mortality risk.

Although serial measurement of serum creatinine over a period of days is an accepted method of detecting and diagnosing AKI and is considered one of the most important tools to evaluate AKI patients, serum creatinine is generally regarded to have several limitations in the diagnosis, assessment and monitoring of AKI patients. The time period for serum creatinine to rise to values (e.g., a 0.3 mg/dL or 25% rise) considered diagnostic for AKI can be 48 hours or longer depending on the definition used. Since cellular injury in AKI can occur over a period of hours, serum creatinine elevations detected at 48 hours or longer can be a late indicator of injury, and relying on serum creatinine can thus delay diagnosis of AKI. Furthermore, serum creatinine is not a good indicator of the exact kidney status and treatment needs during the most acute phases of AKI when kidney function is changing rapidly. Some patients with AKI will recover fully, some will need dialysis (either short term or long term) and some will have other detrimental outcomes including death, major adverse cardiac events and chronic kidney disease. Because serum creatinine is a marker of filtration rate, it does not differentiate between the causes of AKI (pre-renal, intrinsic renal, post-renal obstruction, atheroembolic, etc) or the category or location of injury in intrinsic renal disease (for example, tubular, glomerular or interstitial in origin). Urine output is similarly limited, Knowing these things can be of vital importance in managing and treating patients with AKI.

In addition, Richard Y Lin et al., (1995) "Urinary hyaluronic acid is a Wilms' tumor marker" Journal of pediatric surgery, vol. 30, no. 2, pages 304-308 describe hyaluronic acid as a Wilms' tumor marker. Marino Asselman et al., (2008) "Hyaluronan and Stone Disease" AIP Conference Proceedings, vol. 1049, pages 133-144 deal with the role of hyaluronic acid in stone diseases. Shen Wenqing et al., (2010) "Changes and clinical significance of hyaluronic acid, procollagen type III, collagen type IV and laminin in the serum and urine in patients with chronic renal diseases" Linchuang Yixue (Zhengzhou), CN, vol. 30, no. 6, pages 4-7 describe the detection of hyaluronic acid in patients already suffering from chronic renal disease or uremia. Similarly, Zhang et al., (2006) "Clinical significance of determination of changes of serum hyaluronic acid (HA) and plasma vascular endothelial growth factor (VEGF) contents alter treatment in patients with chronic nephropathy" FANGSHE MIANYIXUE ZAZHI, vol. 19, no. 4, pages 286-28 describe changes in serum hyaluronic acid in patients already afflicted with chronic nephropathy. US patent application 2010/081148 describes a method in which hyaluronic acid is measured in patients, who exhibit an acute renal failure. Honkanen et al., (1991) "Serum hyaluronic acid and procollagen III amino terminal propeptide in chronic renal failure" American Journal Of Nephrology, S. Karger AG, CH, vol. 11, no. 3, pages 201-206 describe serum hyaluronic acid in patients already suffering from chronic renal failure. Hyaluronic acid is also measured in samples obtained from patients receiving hemodialysis. Finally, Haellgren et al., (1987) "Circulating hyaluronate. A potential marker of altered metabolism of the connective tissue in uremia" Nephron Experimental Nephrology, S. Karger AG, CH, vol. 46, no. 2, pages 150-154 measure hyaluronic acid in the serum of patients with already existing renal insufficiency and with end-stage renal failure.

These limitations underscore the need for better methods to detect and assess AKI, particularly in the early and subclinical stages, but also in later stages when recovery and repair of the kidney can occur. Furthermore, there is a need to better identify patients who are at risk of having an AKI.

BRIEF SUMMARY OF THE INVENTION

It is an object of the invention to provide methods and compositions for evaluating renal function in a subject. The object of the invention is solved by the method for evaluating renal status in a subject not receiving renal replacement therapy as defined in claim. The object of the invention is also solved by the use of hyaluronic acid (HA) for the risk stratification as defined in claim 7. Described herein is also the measurement of the kidney injury markers described herein for used for diagnosis, prognosis, risk stratification, staging, monitoring, categorizing and determination of further diagnosis and treatment regimens in subjects suffering or at risk of suffering from an injury to renal function, reduced renal function, and/or acute renal failure (also called acute kidney injury).

Disclosed herein is that these kidney injury markers may be used individually or in panels comprising a plurality of kidney injury markers, for risk stratification (that is, to identify subjects at risk for a future injury to renal function, for future progression to reduced renal function, for future progression to ARF, for future improvement in renal function, etc.); for diagnosis of existing disease (that is, to identify subjects who have suffered an injury to renal function, who have progressed to reduced renal function, who have progressed to ARF, etc.); for monitoring for deterioration or improvement of renal function; and for predicting a future medical outcome, such as improved or worsening renal function, a decreased or increased mortality risk, a decreased or increased risk that a subject will require initiation or continuation of renal replacement therapy (i.e., hemodialysis, peritoneal dialysis, hemofiltration, and/or renal transplantation, a decreased or increased risk that a subject will recover from an injury to renal function, a decreased or increased risk that a subject will recover from ARF, a decreased or increased risk that a subject will progress to end stage renal disease, a decreased or increased risk that a subject will progress to chronic renal failure, a decreased or increased risk that a subject will suffer rejection of a transplanted kidney, etc.

In a first aspect, the present invention relates to a method for evaluating renal status in a subject not receiving renal replacement therapy. This method comprises performing one or more assays configured to detect one or more biomarkers, the biomarkers comprising hyaluronic acid (HA), on a body fluid sample obtained from the subject to provide one or more assay results; and correlating the assay result(s) to one or more of risk stratification to identify subjects at risk for a future injury to renal function, for future progression to reduced renal function, for future progression to ARF and prognosis of the renal status of the subject, wherein the subject is not in acute renal failure and wherein the subject (i) has not experienced a 1.5-fold or greater increase in serum creatinine over a baseline value determined prior to the time at which the body fluid sample is obtained, and/or (iii) has not experienced an increase of 0.3 mg/dL or greater in serum creatinine over a baseline value determined prior to the time at which the body fluid sample is obtained.

In certain embodiments, the methods for evaluating renal status described herein are methods for risk stratification of the subject; that is, assigning a likelihood of one or more future changes in renal status to the subject. In these embodiments, the assay result(s) is/are correlated to one or more such future changes. The following are preferred risk stratification embodiments.

In preferred risk stratification embodiments described herein, these methods comprise determining a subject's risk for a future injury to renal function, and the assay result(s) is/are correlated to a likelihood of such a future injury to renal function. For example, the measured concentration(s) may each be compared to a threshold value. For a "positive going" kidney injury marker, an increased likelihood of suffering a future injury to renal function is assigned to the subject when the measured concentration is above the threshold, relative to a likelihood assigned when the measured concentration is below the threshold. For a "negative going" kidney injury marker, an increased likelihood of suffering a future injury to renal function is assigned to the subject when the measured concentration is below the threshold, relative to a likelihood assigned when the measured concentration is above the threshold.

In other preferred risk stratification embodiments described herein, these methods comprise determining a subject's risk for future reduced renal function, and the assay result(s) is/are correlated to a likelihood of such reduced renal function. For example, the measured concentrations may each be compared to a threshold value. For a "positive going" kidney injury marker, an increased likelihood of suffering a future reduced renal function is assigned to the subject when the measured concentration is above the threshold, relative to a likelihood assigned when the measured concentration is below the threshold. For a "negative going" kidney injury marker, an increased likelihood of future reduced renal function is assigned to the subject when the measured concentration is below the threshold, relative to a likelihood assigned when the measured concentration is above the threshold.

In still other preferred risk stratification embodiments described herein, these methods comprise determining a subject's likelihood for a future improvement in renal function, and the assay result(s) is/are correlated to a likelihood of such a future improvement in renal function. For example, the measured concentration(s) may each be compared to a threshold value. For a "positive going" kidney injury marker, an increased likelihood of a future improvement in renal function is assigned to the subject when the measured concentration is below the threshold, relative to a likelihood assigned when the measured concentration is above the threshold. For a "negative going" kidney injury marker, an increased likelihood of a future improvement in renal function is assigned to the subject when the measured concentration is above the threshold, relative to a likelihood assigned when the measured concentration is below the threshold.

In yet other preferred risk stratification embodiments, these methods described herein comprise determining a subject's risk for progression to ARF, and the result(s) is/are correlated to a likelihood of such progression to ARF. For example, the measured concentration(s) may each be compared to a threshold value. For a "positive going" kidney injury marker, an increased likelihood of progression to ARF is assigned to the subject when the measured concentration is above the threshold, relative to a likelihood assigned when the measured concentration is below the threshold. For a "negative going" kidney injury marker, an increased likelihood of progression to ARF is assigned to the subject when the measured concentration is below the threshold, relative to a likelihood assigned when the measured concentration is above the threshold.

And in other preferred risk stratification embodiments, these methods described herein comprise determining a subject's outcome risk, and the assay result(s) is/are correlated to a likelihood of the occurrence of a clinical outcome related to a renal injury suffered by the subject. For example, the measured concentration(s) may each be compared to a threshold value. For a "positive going" kidney injury marker, an increased likelihood of one or more of: acute kidney injury, progression to a worsening stage of AKI, mortality, a requirement for renal replacement therapy, a requirement for withdrawal of renal toxins, end stage renal disease, heart failure, stroke, myocardial infarction, progression to chronic kidney disease, *etc.,* is assigned to the subject when the measured concentration is above the threshold, relative to a likelihood assigned when the measured concentration is below the threshold. For a "negative going" kidney injury marker, an increased likelihood of one or more of: acute kidney injury, progression to a worsening stage of AKI, mortality, a requirement for renal replacement therapy, a requirement for withdrawal of renal toxins, end stage renal disease, heart failure, stroke, myocardial infarction, progression to chronic kidney disease, *etc.,* is assigned to the subject when the measured concentration is below the threshold, relative to a likelihood assigned when the measured concentration is above the threshold.

In such risk stratification embodiments described herein, preferably the likelihood or risk assigned is that an event of interest is more or less likely to occur within 180 days of the time at which the body fluid sample is obtained from the subject. In particularly preferred embodiments, the likelihood or risk assigned relates to an event of interest occurring within a shorter time period such as 18 months, 120 days, 90 days, 60 days, 45 days, 30 days, 21 days, 14 days, 7 days, 5 days, 96 hours, 72 hours, 48 hours, 36 hours, 24 hours, 12 hours, or less. A risk at 0 hours of the time at which the body fluid sample is obtained from the subject is equivalent to diagnosis of a current condition.

In preferred risk stratification embodiments described herein, the subject is selected for risk stratification based on the pre-existence in the subject of one or more known risk factors for prerenal, intrinsic renal, or postrenal ARF. For example, a subject undergoing or having undergone major vascular surgery, coronary artery bypass, or other cardiac surgery; a subject having pre-existing congestive heart failure, preeclampsia, eclampsia, diabetes mellitus, hypertension, coronary artery disease, proteinuria, renal insufficiency, glomerular filtration below the normal range, cirrhosis, serum creatinine above the normal range, or sepsis; or a subject exposed to NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin are all preferred subjects for monitoring risks according to the methods described herein. This list is not meant to be limiting. By "pre-existence" in this context is meant that the risk factor exists at the time the body fluid sample is obtained from the subject. In particularly preferred embodiments, a subject is chosen for risk stratification based on an existing diagnosis of injury to renal function, reduced renal function, or ARF.

In other embodiments, the methods for evaluating renal status described herein are methods for diagnosing a renal injury in the subject; that is, assessing whether or not a subject has suffered from an injury to renal function, reduced renal function, or ARF. In these embodiments, the assay result(s), for example a measured concentration of HA, is/are correlated to the occurrence or nonoccurrence of a change in renal status. The following are preferred diagnostic embodiments.

In preferred diagnostic embodiments, these methods described herein comprise diagnosing the occurrence or nonoccurrence of an injury to renal function, and the assay result(s) is/are correlated to the occurrence or nonoccurrence of such an injury. For example, each of the measured concentration(s) may be compared to a threshold value. For a positive going marker, an increased likelihood of the occurrence of an injury to renal function is assigned to the subject when the measured concentration is above the threshold (relative to the likelihood assigned when the measured concentration is below the threshold); alternatively, when the measured concentration is below the threshold, an increased likelihood of the nonoccurrence of an injury to renal function may be assigned to the subject (relative to the likelihood assigned when the measured concentration is above the threshold). For a negative going marker, an increased likelihood of the occurrence of an injury to renal function is assigned to the subject when the measured concentration is below the threshold (relative to the likelihood assigned when the measured concentration is above the threshold); alternatively, when the measured concentration is above the threshold, an increased likelihood of the nonoccurrence of an injury to renal function may be assigned to the subject (relative to the likelihood assigned when the measured concentration is below the threshold).

In other preferred diagnostic embodiments, these methods described herein comprise diagnosing the occurrence or nonoccurrence of reduced renal function, and the assay result(s) is/are correlated to the occurrence or nonoccurrence of an injury causing reduced renal function. For example, each of the measured concentration(s) may be compared to a threshold value. For a positive going marker, an increased likelihood of the occurrence of an injury causing reduced renal function is assigned to the subject when the measured concentration is above the threshold (relative to the likelihood assigned when the measured concentration is below the threshold); alternatively, when the measured concentration is below the threshold, an increased likelihood of the nonoccurrence of an injury causing reduced renal function may be assigned to the subject (relative to the likelihood assigned when the measured concentration is above the threshold). For a negative going marker, an increased likelihood of the occurrence of an injury causing reduced renal function is assigned to the subject when the measured concentration is below the threshold (relative to the likelihood assigned when the measured concentration is above the threshold); alternatively, when the measured concentration is above the threshold, an increased likelihood of the nonoccurrence of an injury causing reduced renal function may be assigned to the subject (relative to the likelihood assigned when the measured concentration is below the threshold).

In yet other preferred diagnostic embodiments, these methods described herein comprise diagnosing the occurrence or nonoccurrence of ARF, and the assay result(s) is/are correlated to the occurrence or nonoccurrence of an injury causing ARF. For example, each of the measured concentration(s) may be compared to a threshold value. For a positive going marker, an increased likelihood of the occurrence of ARF is assigned to the subject when the measured concentration is above the threshold (relative to the likelihood assigned when the measured concentration is below the threshold); alternatively, when the measured concentration is below the threshold, an increased likelihood of the nonoccurrence of ARF may be assigned to the subject (relative to the likelihood assigned when the measured concentration is above the threshold). For a negative going marker, an increased likelihood of the occurrence of ARF is assigned to the subject when the measured concentration is below the threshold (relative to the likelihood assigned when the measured concentration is above the threshold); alternatively, when the measured concentration is above the threshold, an increased likelihood of the nonoccurrence of ARF may be assigned to the subject (relative to the likelihood assigned when the measured concentration is below the threshold).

In still other preferred diagnostic embodiments, these methods described herein comprise diagnosing a subject as being in need of renal replacement therapy, and the assay result(s) is/are correlated to a need for renal replacement therapy. For example, each of the measured concentration(s) may be compared to a threshold value. For a positive going marker, an increased likelihood of the occurrence of an injury creating a need for renal replacement therapy is assigned to the subject when the measured concentration is above the threshold (relative to the likelihood assigned when the measured concentration is below the threshold); alternatively, when the measured concentration is below the threshold, an increased likelihood of the nonoccurrence of an injury creating a need for renal replacement therapy may be assigned to the subject (relative to the likelihood assigned when the measured concentration is above the threshold). For a negative going marker, an increased likelihood of the occurrence of an injury creating a need for renal replacement therapy is assigned to the subject when the measured concentration is below the threshold (relative to the likelihood assigned when the measured concentration is above the threshold); alternatively, when the measured concentration is above the threshold, an increased likelihood of the nonoccurrence of an injury creating a need for renal replacement therapy may be assigned to the subject (relative to the likelihood assigned when the measured concentration is below the threshold).

In still other preferred diagnostic embodiments, these methods comprise diagnosing a subject as being in need of renal transplantation, and the assay result(s) is/are correlated to a need for renal transplantation. For example, each of the measured concentration(s) may be compared to a threshold value. For a positive going marker, an increased likelihood of the occurrence of an injury creating a need for renal transplantation is assigned to the subject when the measured concentration is above the threshold (relative to the likelihood assigned when the measured concentration is below the threshold); alternatively, when the measured concentration is below the threshold, an increased likelihood of the nonoccurrence of an injury creating a need for renal transplantation may be assigned to the subject (relative to the likelihood assigned when the measured concentration is above the threshold). For a negative going marker, an increased likelihood of the occurrence of an injury creating a need for renal transplantation is assigned to the subject when the measured concentration is below the threshold (relative to the likelihood assigned when the measured concentration is above the threshold); alternatively, when the measured concentration is above the threshold, an increased likelihood of the nonoccurrence of an injury creating a need for renal transplantation may be assigned to the subject (relative to the likelihood assigned when the measured concentration is below the threshold).

In still other embodiments, the methods for evaluating renal status described herein are methods for monitoring a renal injury in the subject; that is, assessing whether or not renal function is improving or worsening in a subject who has suffered from an injury to renal function, reduced renal function, or ARF. In these embodiments, the assay result(s), for example a measured concentration of HA, is/are correlated to the occurrence or nonoccurrence of a change in renal status. The following are preferred monitoring embodiments.

In preferred monitoring embodiments, these methods described herein comprise monitoring renal status in a subject suffering from an injury to renal function, and the assay result(s) is/are correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In other preferred monitoring embodiments, these methods comprise monitoring renal status in a subject suffering from reduced renal function, and the assay result(s) is/are correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In yet other preferred monitoring embodiments, these methods described herein comprise monitoring renal status in a subject suffering from acute renal failure, and the assay result(s) is/are correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In other additional preferred monitoring embodiments, these methods described herein comprise monitoring renal status in a subject at risk of an injury to renal function due to the pre-existence of one or more known risk factors for prerenal, intrinsic renal, or postrenal ARF, and the assay result(s) is/are correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In still other embodiments, the methods for evaluating renal status described herein are methods for classifying a renal injury in the subject; that is, determining whether a renal injury in a subject is prerenal, intrinsic renal, or postrenal; and/or further subdividing these classes into subclasses such as acute tubular injury, acute glomerulonephritis acute tubulointerstitial nephritis, acute vascular nephropathy, or infiltrative disease; and/or assigning a likelihood that a subject will progress to a particular RIFLE stage. In these embodiments, the assay result(s), for example a measured concentration of HA, is/are correlated to a particular class and/or subclass. The following are preferred classification embodiments.

In preferred classification embodiments, these methods described herein comprise determining whether a renal injury in a subject is prerenal, intrinsic renal, or postrenal; and/or further subdividing these classes into subclasses such as acute tubular injury, acute glomerulonephritis acute tubulointerstitial nephritis, acute vascular nephropathy, or infiltrative disease; and/or assigning a likelihood that a subject will progress to a particular RIFLE stage, and the assay result(s) is/are correlated to the injury classification for the subject. For example, the measured concentration may be compared to a threshold value, and when the measured concentration is above the threshold, a particular classification is assigned; alternatively, when the measured concentration is below the threshold, a different classification may be assigned to the subject.

A variety of methods may be used by the skilled artisan to arrive at a desired threshold value for use in these methods. For example, the threshold value may be determined from a population of normal subjects by selecting a concentration representing the 75th, 85th, 90th, 95th, or 99th percentile of a kidney injury marker measured in such normal subjects. Alternatively, the threshold value may be determined from a "diseased" population of subjects, e.g., those suffering from an injury or having a predisposition for an injury (e.g., progression to ARF or some other clinical outcome such as death, dialysis, renal transplantation, *etc.),* by selecting a concentration representing the 75th, 85th, 90th, 95th, or 99th percentile of a kidney injury marker measured in such subjects. In another alternative, the threshold value may be determined from a prior measurement of a kidney injury marker in the same subject; that is, a temporal change in the level of a kidney injury marker in the subject may be used to assign risk to the subject.

The foregoing discussion is not meant to imply, however, that the kidney injury markers of the present invention must be compared to corresponding individual thresholds. Methods for combining assay results can comprise the use of multivariate logistical regression, loglinear modeling, neural network analysis, n-of-m analysis, decision tree analysis, calculating ratios of markers, *etc.* This list is not meant to be limiting. In these methods, a composite result which is determined by combining individual markers may be treated as if it is itself a marker; that is, a threshold may be determined for the composite result as described herein for individual markers, and the composite result for an individual patient compared to this threshold.

The ability of a particular test to distinguish two populations can be established using ROC analysis. For example, ROC curves established from a "first" subpopulation which is predisposed to one or more future changes in renal status, and a "second" subpopulation which is not so predisposed can be used to calculate a ROC curve, and the area under the curve provides a measure of the quality of the test. Preferably, the tests described herein provide a ROC curve area greater than 0.5, preferably at least 0.6, more preferably 0.7, still more preferably at least 0.8, even more preferably at least 0.9, and most preferably at least 0.95.

In certain aspects, the measured concentration of one or more kidney injury markers, or a composite of such markers, may be treated as continuous variables. For example, any particular concentration can be converted into a corresponding probability of a future reduction in renal function for the subject, the occurrence of an injury, a classification, etc. In yet another alternative, a threshold that can provide an acceptable level of specificity and sensitivity in separating a population of subjects into "bins" such as a "first" subpopulation (e.g., which is predisposed to one or more future changes in renal status, the occurrence of an injury, a classification, etc.) and a "second" subpopulation which is not so predisposed. A threshold value is selected to separate this first and second population by one or more of the following measures of test accuracy:

an odds ratio greater than 1, preferably at least about 2 or more or about 0.5 or less, more preferably at least about 3 or more or about 0.33 or less, still more preferably at least about 4 or more or about 0.25 or less, even more preferably at least about 5 or more or about 0.2 or less, and most preferably at least about 10 or more or about 0.1 or less;

a specificity of greater than 0.5, preferably at least about 0.6, more preferably at least about 0.7, still more preferably at least about 0.8, even more preferably at least about 0.9 and most preferably at least about 0.95, with a corresponding sensitivity greater than 0.2, preferably greater than about 0.3, more preferably greater than about 0.4, still more preferably at least about 0.5, even more preferably about 0.6, yet more preferably greater than about 0.7, still more preferably greater than about 0.8, more preferably greater than about 0.9, and most preferably greater than about 0.95;

a sensitivity of greater than 0.5, preferably at least about 0.6, more preferably at least about 0.7, still more preferably at least about 0.8, even more preferably at least about 0.9 and most preferably at least about 0.95, with a corresponding specificity greater than 0.2, preferably greater than about 0.3, more preferably greater than about 0.4, still more preferably at least about 0.5, even more preferably about 0.6, yet more preferably greater than about 0.7, still more preferably greater than about 0.8, more preferably greater than about 0.9, and most preferably greater than about 0.95;

at least about 75% sensitivity, combined with at least about 75% specificity;

a positive likelihood ratio (calculated as sensitivity/(1-specificity)) of greater than 1, at least about 2, more preferably at least about 3, still more preferably at least about 5, and most preferably at least about 10; or

a negative likelihood ratio (calculated as (1-sensitivity)/specificity) of less than 1, less than or equal to about 0.5, more preferably less than or equal to about 0.3, and most preferably less than or equal to about 0.1.

The term "about" in the context of any of the above measurements refers to +/- 5% of a given measurement.

Multiple thresholds may also be used to assess renal status in a subject. For example, a "first" subpopulation which is predisposed to one or more future changes in renal status, the occurrence of an injury, a classification, etc., and a "second" subpopulation which is not so predisposed can be combined into a single group. This group is then subdivided into three or more equal parts (known as tertiles, quartiles, quintiles, etc., depending on the number of subdivisions). An odds ratio is assigned to subjects based on which subdivision they fall into. If one considers a tertile, the lowest or highest tertile can be used as a reference for comparison of the other subdivisions. This reference subdivision is assigned an odds ratio of 1. The second tertile is assigned an odds ratio that is relative to that first tertile. That is, someone in the second tertile might be 3 times more likely to suffer one or more future changes in renal status in comparison to someone in the first tertile. The third tertile is also assigned an odds ratio that is relative to that first tertile.

In certain embodiments, the assay method is an immunoassay. Antibodies for use in such assays will specifically bind a full length kidney injury marker of interest, and may also bind one or more polypeptides that are "related" thereto, as that term is defined hereinafter. Numerous immunoassay formats are known to those of skill in the art. Preferred body fluid samples are selected from the group consisting of urine, blood, serum, saliva, tears, and plasma.

The foregoing method steps should not be interpreted to mean that the kidney injury marker assay result(s) is/are used in isolation in the methods described herein. Rather, additional variables or other clinical indicia may be included in the methods described herein. For example, a risk stratification, diagnostic, classification, monitoring, etc. method described or claimed herein may combine the assay result(s) with one or more variables measured for the subject selected from the group consisting of demographic information (e.g., weight, sex, age, race), medical history (e.g., family history, type of surgery, pre-existing disease such as aneurism, congestive heart failure, preeclampsia, eclampsia, diabetes mellitus, hypertension, coronary artery disease, proteinuria, renal insufficiency, or sepsis, type of toxin exposure such as NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin), clinical variables (e.g., blood pressure, temperature, respiration rate), risk scores (APACHE score, PREDICT score, TIMI Risk Score for UA/NSTEMI, Framingham Risk Score), a glomerular filtration rate, an estimated glomerular filtration rate, a urine production rate, a serum or plasma creatinine concentration, a urine creatinine concentration, a fractional excretion of sodium, a urine sodium concentration, a urine creatinine to serum or plasma creatinine ratio, a urine specific gravity, a urine osmolality, a urine urea nitrogen to plasma urea nitrogen ratio, a plasma BUN to creatnine ratio, a renal failure index calculated as urine sodium / (urine creatinine / plasma creatinine), a serum or plasma neutrophil gelatinase (NGAL) concentration, a urine NGAL concentration, a serum or plasma cystatin C concentration, a serum or plasma cardiac troponin concentration, a serum or plasma BNP concentration, a serum or plasma NTproBNP concentration, and a serum or plasma proBNP concentration. Other measures of renal function which may be combined with one or more kidney injury marker assay result(s) are described hereinafter and in Harrison's Principles of Internal Medicine, 17th Ed., McGraw Hill, New York, pages 1741-1830, and Current Medical Diagnosis & Treatment 2008, 47th Ed, McGraw Hill, New York, pages 785-815.

When more than one marker is measured, the individual markers may be measured in samples obtained at the same time, or may be determined from samples obtained at different (e.g., an earlier or later) times. The individual markers may also be measured on the same or different body fluid samples. For example, one kidney injury marker may be measured in a serum or plasma sample and another kidney injury marker may be measured in a urine sample. In addition, assignment of a likelihood may combine an individual kidney injury marker assay result with temporal changes in one or more additional variables.

In various related aspects, disclosed herein are devices and kits for performing the methods described herein. Suitable kits comprise reagents sufficient for performing an assay for at least one of the described kidney injury markers, together with instructions for performing the described threshold comparisons.

In certain embodiments, reagents for performing such assays are provided in an assay device, and such assay devices may be included in such a kit. Preferred reagents can comprise one or more solid phase antibodies, the solid phase antibody comprising antibody that detects the intended biomarker target(s) bound to a solid support. In the case of sandwich immunoassays, such reagents can also include one or more detectably labeled antibodies, the detectably labeled antibody comprising antibody that detects the intended biomarker target(s) bound to a detectable label. Additional optional elements that may be provided as part of an assay device are described hereinafter.

Detectable labels may include molecules that are themselves detectable (e.g., fluorescent moieties, electrochemical labels, eel (electrochemical luminescence) labels, metal chelates, colloidal metal particles, *etc.*) as well as molecules that may be indirectly detected by production of a detectable reaction product (e.g., enzymes such as horseradish peroxidase, alkaline phosphatase, *etc.)* or through the use of a specific binding molecule which itself may be detectable (e.g., a labeled antibody that binds to the second antibody, biotin, digoxigenin, maltose, oligohistidine, 2,4-dintrobenzene, phenylarsenate, ssDNA, dsDNA, *etc.*).

Generation of a signal from the signal development element can be performed using various optical, acoustical, and electrochemical methods well known in the art. Examples of detection modes include fluorescence, radiochemical detection, reflectance, absorbance, amperometry, conductance, impedance, interferometry, ellipsometry, *etc.* In certain of these methods, the solid phase antibody is coupled to a transducer (*e.g.*, a diffraction grating, electrochemical sensor, etc) for generation of a signal, while in others, a signal is generated by a transducer that is spatially separate from the solid phase antibody (*e.g.*, a fluorometer that employs an excitation light source and an optical detector). This list is not meant to be limiting. Antibody-based biosensors may also be employed to determine the presence or amount of analytes that optionally eliminate the need for a labeled molecule.

## BRIEF DESCRIPTION OF THE FIGURES

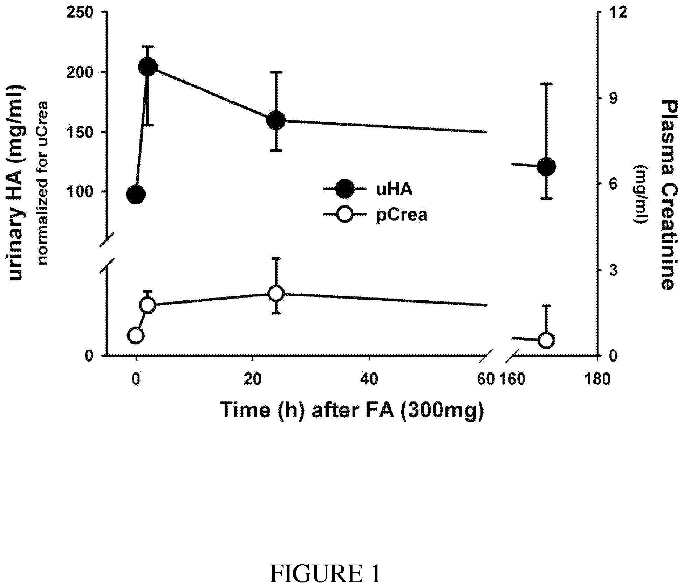

Fig. 1 depicts the change in normalized urinary concentration of hyaluronic acid in response to a chemically induced acute kidney injury.

## DETAILED DESCRIPTION OF THE INVENTION

The present invention relates to a method as defined in claim 1 and to a use as defined in claim 7. Disclosed herein are also compositions for diagnosis, differential diagnosis, risk stratification, monitoring, classifying and determination of treatment regimens in subjects suffering or at risk of suffering from injury to renal function, reduced renal function and/or acute renal failure through measurement of one or more kidney injury markers of the present invention.

The following is a brief description of the kidney injury marker Hyaluronic acid (HA) that is used in the present invention.

Hyaluronic acid (HA) is a ubiquitous connective tissue glycosaminoglycan that in vivo is present as a high molecular mass component of most extracellular matrices. Although HA is not a major constituent of the normal renal corticointerstitium,[3] it is expressed around renal proximal tubular epithelial cells (PTC) after both acute and chronic renal injury that is caused by numerous diseases.[4, 5] Furthermore, increased deposition of interstitial HA correlates with both proteinuria and renal function in progressive renal disease.[6] Binding of HA to its principle receptor, CD44, promotes inflammation through interaction between HA and CD44, expressed on inflammatory cells.[7] HA/CD44 binding activates the mitogen-activated protein kinase (MAPK) pathway and enhances PTC migration, a process that is implicated in epithelial cell-fibroblast transdifferentiation and progressive renal fibrosis.[8] In ischemic kidneys from diabetic subjects, the renal HA-content started to increases already after 24 hours and significantly so 1-8 weeks after ischemia/reperfusion (I/R).[9]

For purposes of this document, the following definitions apply:

As used herein, an "injury to renal function" is an abrupt (within 14 days, preferably within 7 days, more preferably within 72 hours, and still more preferably within 48 hours) measurable reduction in a measure of renal function. Such an injury may be identified, for example, by a decrease in glomerular filtration rate or estimated GFR, a reduction in urine output, an increase in serum creatinine, an increase in serum cystatin C, a requirement for renal replacement therapy, etc. "Improvement in Renal Function" is an abrupt (within 14 days, preferably within 7 days, more preferably within 72 hours, and still more preferably within 48 hours) measurable increase in a measure of renal function. Preferred methods for measuring and/or estimating GFR are described hereinafter.

As used herein, "reduced renal function" is an abrupt (within 14 days, preferably within 7 days, more preferably within 72 hours, and still more preferably within 48 hours) reduction in kidney function identified by an absolute increase in serum creatinine of greater than or equal to 0.1 mg/dL ($\geq$ 8.8 $\mu$mol/L), a percentage increase in serum creatinine of greater than or equal to 20% (1.2-fold from baseline), or a reduction in urine output (documented oliguria of less than 0.5 ml/kg per hour).

As used herein, "acute renal failure" or "ARF" is an abrupt (within 14 days, preferably within 7 days, more preferably within 72 hours, and still more preferably within 48 hours) reduction in kidney function identified by an absolute increase in serum creatinine of greater than or equal to 0.3 mg/dl ($\geq$ 26.4 $\mu$mol/l), a percentage increase in serum creatinine of greater than or equal to 50% (1. 5-fold from baseline), or a reduction in urine output (documented oliguria of less than 0.5 ml/kg per hour for at least 6 hours). This term is synonymous with "acute kidney injury" or "AKI."

In this regard, the skilled artisan will understand that the signals obtained from an immunoassay are a direct result of complexes formed between one or more antibodies and the target biomolecule (*i.e.*, the analyte) and polypeptides containing the necessary epitope(s) to which the antibodies bind. While such assays may detect the full length biomarker and the assay result be expressed as a concentration of a biomarker of interest, the signal from the assay is actually a result of all such "immunoreactive" polypeptides present in the sample. Expression of biomarkers may also be determined by means other than immunoassays, including protein measurements (such as dot blots, western blots, chromatographic methods, mass spectrometry, *etc.)* and nucleic acid measurements (mRNA quantitation). This list is not meant to be limiting.

As used herein, the term "relating a signal to the presence or amount" of an analyte reflects this understanding. Assay signals are typically related to the presence or amount of an analyte through the use of a standard curve calculated using known concentrations of the analyte of interest. The skilled artisan will understand that the signals obtained from an assay are often a direct result of complexes formed between one or more antibodies and the target biomolecule (i.e., the analyte) and polypeptides containing the necessary epitope(s) to which the antibodies bind. While such assays may detect the full length biomarker and the assay result be expressed as a concentration of a biomarker of interest, the signal from the assay is actually a result of all such "immunoreactive" polypeptides present in the sample. Expression of biomarkers may also be determined by means other than immunoassays, including protein measurements (such as dot blots, western blots, chromatographic methods, mass spectrometry, etc.) and nucleic acid measurements (mRNA quantitation). This list is not meant to be limiting.

As the term is used herein, an assay is "configured to detect" an analyte if an assay can generate a detectable signal indicative of the presence or amount of a physiologically relevant concentration of the analyte. Because an antibody epitope is on the order of 8 amino acids, an immunoassay configured to detect a marker of interest will also detect polypeptides related to the marker sequence, so long as those polypeptides contain the epitope(s) necessary to bind to the antibody or antibodies used in the assay. The term "related marker" as used herein with regard to a biomarker such as one of the kidney injury markers described herein refers to one or more fragments, variants, etc., of a particular marker or its biosynthetic parent that may be detected as a surrogate for the marker itself or as independent biomarkers. The term also refers to one or more polypeptides present in a biological sample that are derived from the biomarker precursor complexed to additional species, such as binding proteins, receptors, heparin, lipids, sugars, *etc.*

The term "positive going" marker as that term is used herein refer to a marker that is determined to be elevated in subjects suffering from a disease or condition, relative to subjects not suffering from that disease or condition. The term "negative going" marker as that term is used herein refer to a marker that is determined to be reduced in subjects suffering from a disease or condition, relative to subjects not suffering from that disease or condition.

The term "subject" as used herein refers to a human or non-human organism. Thus, the methods and compositions described herein are applicable to both human and veterinary disease. Further, while a subject is preferably a living organism, the invention described herein may be used in post-mortem analysis as well. Preferred subjects are humans, and most preferably "patients," which as used herein refers to living humans that are receiving medical care for a disease or condition. This includes persons with no defined illness who are being investigated for signs of pathology.

Preferably, an analyte is measured in a sample. Such a sample may be obtained from a subject, or may be obtained from biological materials intended to be provided to the subject. For example, a sample may be obtained from a kidney being evaluated for possible transplantation into a subject, and an analyte measurement used to evaluate the kidney for preexisting damage. Preferred samples are body fluid samples.

The term "body fluid sample" as used herein refers to a sample of bodily fluid obtained for the purpose of diagnosis, prognosis, classification or evaluation of a subject of interest, such as a patient or transplant donor. In certain embodiments, such a sample may be obtained for the purpose of determining the outcome of an ongoing condition or the effect of a treatment regimen on a condition. Preferred body fluid samples include blood, serum, plasma, cerebrospinal fluid, urine, saliva, sputum, and pleural effusions. In addition, one of skill in the art would realize that certain body fluid samples would be more readily analyzed following a fractionation or purification procedure, for example, separation of whole blood into serum or plasma components.

The term "diagnosis" as used herein refers to methods by which the skilled artisan can estimate and/or determine the probability ("a likelihood") of whether or not a patient is suffering from a given disease or condition. In the case of the present invention, "diagnosis" includes using the results of an assay, most preferably an immunoassay, for a kidney injury marker of the present invention, optionally together with other clinical characteristics, to arrive at a diagnosis (that is, the occurrence or nonoccurrence) of an acute renal injury or ARF for the subject from which a sample was obtained and assayed. That such a diagnosis is "determined" is not meant to imply that the diagnosis is 100% accurate. Many biomarkers are indicative of multiple conditions. The skilled clinician does not use biomarker results in an informational vacuum, but rather test results are used together with other clinical indicia to arrive at a diagnosis. Thus, a measured biomarker level on one side of a predetermined diagnostic threshold indicates a greater likelihood of the occurrence of disease in the subject relative to a measured level on the other side of the predetermined diagnostic threshold.

Similarly, a prognostic risk signals a probability ("a likelihood") that a given course or outcome will occur. A level or a change in level of a prognostic indicator, which in turn is associated with an increased probability of morbidity (e.g., worsening renal function, future ARF, or death) is referred to as being "indicative of an increased likelihood" of an adverse outcome in a patient.

Marker Assays

In general, immunoassays involve contacting a sample containing or suspected of containing a biomarker of interest with at least one antibody that specifically binds to the biomarker. A signal is then generated indicative of the presence or amount of complexes formed by the binding of polypeptides in the sample to the antibody. The signal is then related to the presence or amount of the biomarker in the sample. Numerous methods and devices are well known to the skilled artisan for the detection and analysis of biomarkers. See, e.g., U.S. Patents 6,143,576; 6,113,855; 6,019,944; 5,985,579; 5,947,124; 5,939,272; 5,922,615; 5,885,527; 5,851,776; 5,824,799; 5,679,526; 5,525,524; and 5,480,792, and The Immunoassay Handbook, David Wild, ed. Stockton Press, New York, 1994.

The assay devices and methods known in the art can utilize labeled molecules in various sandwich, competitive, or non-competitive assay formats, to generate a signal that is related to the presence or amount of the biomarker of interest. Suitable assay formats also include chromatographic, mass spectrographic, and protein "blotting" methods. Additionally, certain methods and devices, such as biosensors and optical immunoassays, may be employed to determine the presence or amount of analytes without the need for a labeled molecule. See, e.g., U.S. Patents 5,631,171; and 5,955,377. One skilled in the art also recognizes that robotic instrumentation including but not limited to Beckman ACCESS®, Abbott AXSYM®, Roche ELECSYS®, Dade Behring STRATUS® systems are among the immunoassay analyzers that are capable of performing immunoassays. But any suitable immunoassay may be utilized, for example, enzyme-linked immunoassays (ELISA), radioimmunoassays (RIAs), competitive binding assays, and the like.

Antibodies or other polypeptides may be immobilized onto a variety of solid supports for use in assays. Solid phases that may be used to immobilize specific binding members include those developed and/or used as solid phases in solid phase binding assays. Examples of suitable solid phases include membrane filters, cellulose-based papers, beads (including polymeric, latex and paramagnetic particles), glass, silicon wafers, microparticles, nanoparticles, TentaGels, AgroGels, PEGA gels, SPOCC gels, and multiple-well plates. An assay strip could be prepared by coating the antibody or a plurality of antibodies in an array on solid support. This strip could then be dipped into the test sample and then processed quickly through washes and detection steps to generate a measurable signal, such as a colored spot. Antibodies or other polypeptides may be bound to specific zones of assay devices either by conjugating directly to an assay device surface, or by indirect binding. In an example of the later case, antibodies or other polypeptides may be immobilized on particles or other solid supports, and that solid support immobilized to the device surface.

Biological assays require methods for detection, and one of the most common methods for quantitation of results is to conjugate a detectable label to a protein or nucleic acid that has affinity for one of the components in the biological system being studied. Detectable labels may include molecules that are themselves detectable (e.g., fluorescent moieties, electrochemical labels, metal chelates, etc.) as well as molecules that may be indirectly detected by production of a detectable reaction product (e.g., enzymes such as horseradish peroxidase, alkaline phosphatase, etc.) or by a specific binding molecule which itself may be detectable (e.g., biotin, digoxigenin, maltose, oligohistidine, 2,4-dintrobenzene, phenylarsenate, ssDNA, dsDNA, etc.).

Preparation of solid phases and detectable label conjugates often comprise the use of chemical cross-linkers. Cross-linking reagents contain at least two reactive groups, and are divided generally into homofunctional cross-linkers (containing identical reactive groups) and heterofunctional cross-linkers (containing non-identical reactive groups). Homobifunctional cross-linkers that couple through amines, sulfhydryls or react non-specifically are available from many commercial sources. Maleimides, alkyl and aryl halides, alpha-haloacyls and pyridyl disulfides are thiol reactive groups. Maleimides, alkyl and aryl halides, and alpha-haloacyls react with sulfhydryls to form thiol ether bonds, while pyridyl disulfides react with sulfhydryls to produce mixed disulfides. The pyridyl disulfide product is cleavable. Imidoesters are also very useful for protein-protein cross-links. A variety of heterobifunctional cross-linkers, each combining different attributes for successful conjugation, are commercially available.

In certain aspects disclosed herein are kits for the analysis of the described kidney injury markers. The kit comprises reagents for the analysis of at least one test sample which comprise at least one antibody that a kidney injury marker. The kit can also include devices and instructions for performing one or more of the diagnostic and/or prognostic correlations described herein. Preferred kits will comprise an antibody pair for performing a sandwich assay, or a labeled species for performing a competitive assay, for the analyte. Preferably, an antibody pair comprises a first antibody conjugated to a solid phase and a second antibody conjugated to a detectable label, wherein each of the first and second antibodies that bind a kidney injury marker. Most preferably each of the antibodies are monoclonal antibodies. The instructions for use of the kit and performing the correlations can be in the form of labeling, which refers to any written or recorded material that is attached to, or otherwise accompanies a kit at any time during its manufacture, transport, sale or use. For example, the term labeling encompasses advertising leaflets and brochures, packaging materials, instructions, audio or video cassettes, computer discs, as well as writing imprinted directly on kits.

Antibodies

The term "antibody" as used herein refers to a peptide or polypeptide derived from, modeled after or substantially encoded by an immunoglobulin gene or immunoglobulin genes, or fragments thereof, capable of specifically binding an antigen or epitope. *See, e.g.* Fundamental Immunology, 3rd Edition, W.E. Paul, ed., Raven Press, N.Y. (1993); Wilson (1994; J. Immunol. Methods 175:267-273; Yarmush (1992) J. Biochem. Biophys. Methods 25:85-97. The term antibody includes antigen-binding portions, i.e., "antigen binding sites," (e.g., fragments, subsequences, complementarity determining regions (CDRs)) that retain capacity to bind antigen, including (i) a Fab fragment, a monovalent fragment consisting of the VL, VH, CL and CHl domains; (ii) a F(ab')2 fragment, a bivalent fragment comprising two Fab fragments linked by a disulfide bridge at the hinge region; (iii) a Fd fragment consisting of the VH and CHl domains; (iv) a Fv fragment consisting of the VL and VH domains of a single arm of an antibody, (v) a dAb fragment (Ward et al., (1989) Nature 341:544-546), which consists of a VH domain; and (vi) an isolated complementarity determining region (CDR). Single chain antibodies are also included by reference in the term "antibody."

Antibodies used in the immunoassays described herein preferably specifically bind to a kidney injury marker of the present invention. The term "specifically binds" is not intended to indicate that an antibody binds exclusively to its intended target since, as noted above, an antibody binds to any polypeptide displaying the epitope(s) to which the antibody binds. Rather, an antibody "specifically binds" if its affinity for its intended target is about 5-fold greater when compared to its affinity for a non-target molecule which does not display the appropriate epitope(s). Preferably the affinity of the antibody will be at least about 5 fold, preferably 10 fold, more preferably 25-fold, even more preferably 50-fold, and most preferably 100-fold or more, greater for a target molecule than its affinity for a non-target molecule. In preferred embodiments, Preferred antibodies bind with affinities of at least about $10^7$ M$^{-1}$, and preferably between about $10^8$ M$^{-1}$ to about $10^9$ M$^{-1}$, about $10^9$ M$^{-1}$ to about $10^{10}$ M$^{-1}$, or about $10^{10}$ M$^{-1}$ to about $10^{12}$ M$^{-1}$.

Affinity is calculated as $K_d = k_{off}/k_{on}$ ($k_{off}$ is the dissociation rate constant, $K_{on}$ is the association rate constant and $K_d$ is the equilibrium constant). Affinity can be determined at equilibrium by measuring the fraction bound (r) of labeled ligand at various concentrations (c). The data are graphed using the Scatchard equation: r/c = K(n-r): where r = moles of bound ligand/mole of receptor at equilibrium; c = free ligand concentration at equilibrium; K = equilibrium association constant; and n = number of ligand binding sites per receptor molecule. By graphical analysis, r/c is plotted on the Y-axis versus r on the X-axis, thus producing a Scatchard plot. Antibody affinity measurement by Scatchard analysis is well known in the art. *See, e.g.,* van Erp et al., J. Immunoassay 12: 425-43, 1991; Nelson and Griswold, Comput. Methods Program Biomed. 27: 65-8, 1988.

The term "epitope" refers to an antigenic determinant capable of specific binding to an antibody. Epitopes usually consist of chemically active surface groupings of molecules such as amino acids or sugar side chains and usually have specific three dimensional structural characteristics, as well as specific charge characteristics. Conformational and nonconformational epitopes are distinguished in that the binding to the former but not the latter is lost in the presence of denaturing solvents.

Numerous publications discuss the use of phage display technology to produce and screen libraries of polypeptides for binding to a selected analyte. *See, e.g,* Cwirla et al., Proc. Natl. Acad. Sci. USA 87, 6378-82, 1990; Devlin et al., Science 249, 404-6, 1990, Scott and Smith, Science 249, 386-88, 1990; and Ladner et al., U.S. Pat. No. 5,571,698. A basic concept of phage display methods is the establishment of a physical association between DNA encoding a polypeptide to be screened and the polypeptide. This physical association is provided by the phage particle, which displays a polypeptide as part of a capsid enclosing the phage genome which encodes the polypeptide. The establishment of a physical association between polypeptides and their genetic material allows simultaneous mass screening of very large numbers of phage bearing different polypeptides. Phage displaying a polypeptide with affinity to a target bind to the target and these phage are enriched by affinity screening to the target. The identity of polypeptides displayed from these phage can be determined from their respective genomes. Using these methods a polypeptide identified as having a binding affinity for a desired target can then be synthesized in bulk by conventional means. *See, e.g.,* U.S. Patent No. 6,057,098.

The antibodies that are generated by these methods may then be selected by first screening for affinity and specificity with the purified polypeptide of interest and, if required, comparing the results to the affinity and specificity of the antibodies with polypeptides that are desired to be excluded from binding. The screening procedure can involve immobilization of the purified polypeptides in separate wells of microtiter plates. The solution containing a potential antibody or groups of antibodies is then placed into the respective microtiter wells and incubated for about 30 min to 2 h. The microtiter wells are then washed and a labeled secondary antibody (for example, an anti-mouse antibody conjugated to alkaline phosphatase if the raised antibodies are mouse antibodies) is added to the wells and incubated for about 30 min and then washed. Substrate is added to the wells and a color reaction will appear where antibody to the immobilized polypeptide(s) are present.

The antibodies so identified may then be further analyzed for affinity and specificity in the assay design selected. In the development of immunoassays for a target protein, the purified target protein acts as a standard with which to judge the sensitivity and specificity of the immunoassay using the antibodies that have been selected. Because the binding affinity of various antibodies may differ; certain antibody pairs (*e.g.,* in sandwich assays) may interfere with one another sterically, *etc.,* assay performance of an antibody may be a more important measure than absolute affinity and specificity of an antibody.

Assay Correlations

The term "correlating" as used herein in reference to the use of biomarkers refers to comparing the presence or amount of the biomarker(s) in a patient to its presence or amount in persons known to suffer from, or known to be at risk of, a given condition; or in persons known to be free of a given condition. Often, this takes the form of comparing an assay result in the form of a biomarker concentration to a predetermined threshold selected to be indicative of the occurrence or nonoccurrence of a disease or the likelihood of some future outcome.

Selecting a diagnostic threshold involves, among other things, consideration of the probability of disease, distribution of true and false diagnoses at different test thresholds, and estimates of the consequences of treatment (or a failure to treat) based on the diagnosis. For example, when considering administering a specific therapy which is highly efficacious and has a low level of risk, few tests are needed because clinicians can accept substantial diagnostic uncertainty. On the other hand, in situations where treatment options are less effective and more risky, clinicians often need a higher degree of diagnostic certainty. Thus, cost/benefit analysis is involved in selecting a diagnostic threshold.

Suitable thresholds may be determined in a variety of ways. For example, one recommended diagnostic threshold for the diagnosis of acute myocardial infarction using cardiac troponin is the $97.5^{th}$ percentile of the concentration seen in a normal population. Another method may be to look at serial samples from the same patient, where a prior "baseline" result is used to monitor for temporal changes in a biomarker level.

Population studies may also be used to select a decision threshold. Reciever Operating Characteristic ("ROC") arose from the field of signal dectection therory developed during World War II for the analysis of radar images, and ROC analysis is often used to select a threshold able to best distinguish a "diseased" subpopulation from a "nondiseased" subpopulation. A false positive in this case occurs when the person tests positive, but actually does not have the disease. A false negative, on the other hand, occurs when the person tests negative, suggesting they are healthy, when they actually do have the disease. To draw a ROC curve, the true positive rate (TPR) and false positive rate (FPR) are determined as the decision threshold is varied continuously. Since TPR is equivalent with sensitivity and FPR is equal to 1 - specificity, the ROC graph is sometimes called the sensitivity vs (1 - specificity) plot. A perfect test will have an area under the ROC curve of 1.0; a random test will have an area of 0.5. A threshold is selected to provide an acceptable level of specificity and sensitivity.

In this context, "diseased" is meant to refer to a population having one characteristic (the presence of a disease or condition or the occurrence of some outcome) and "nondiseased" is meant to refer to a population lacking the characteristic. While a single decision threshold is the simplest application of such a method, multiple decision thresholds may be used. For example, below a first threshold, the absence of disease may be assigned with relatively high confidence, and above a second threshold the presence of disease may also be assigned with relatively high confidence. Between the two thresholds may be considered indeterminate. This is meant to be exemplary in nature only.

In addition to threshold comparisons, other methods for correlating assay results to a patient classification (occurrence or nonoccurrence of disease, likelihood of an outcome, *etc.)* include decision trees, rule sets, Bayesian methods, and neural network methods. These methods can produce probability values representing the degree to which a subject belongs to one classification out of a plurality of classifications.

Measures of test accuracy may be obtained as described in Fischer et al., Intensive Care Med. 29: 1043-51, 2003, and used to determine the effectiveness of a given biomarker. These measures include sensitivity and specificity, predictive values, likelihood ratios, diagnostic odds ratios, and ROC curve areas. The area under the curve ("AUC") of a ROC plot is equal to the probability that a classifier will rank a randomly chosen positive instance higher than a randomly chosen negative one. The area under the ROC curve may be thought of as equivalent to the Mann-Whitney U test, which tests for the median difference between scores obtained in the two groups considered if the groups are of continuous data, or to the Wilcoxon test of ranks.

As discussed above, suitable tests may exhibit one or more of the following results on these various measures: a specificity of greater than 0.5, preferably at least 0.6, more preferably at least 0.7, still more preferably at least 0.8, even more preferably at least 0.9 and most preferably at least 0.95, with a corresponding sensitivity greater than 0.2, preferably greater than 0.3, more preferably greater than 0.4, still more preferably at least 0.5, even more preferably 0.6, yet more preferably greater than 0.7, still more preferably greater than 0.8, more preferably greater than 0.9, and most preferably greater than 0.95; a sensitivity of greater than 0.5, preferably at least 0.6, more preferably at least 0.7, still more preferably at least 0.8, even more preferably at least 0.9 and most preferably at least 0.95, with a corresponding specificity greater than 0.2, preferably greater than 0.3, more preferably greater than 0.4, still more preferably at least 0.5, even more preferably 0.6, yet more preferably greater than 0.7, still more preferably greater than 0.8, more preferably greater than 0.9, and most preferably greater than 0.95; at least 75% sensitivity, combined with at least 75% specificity; a ROC curve area of greater than 0.5, preferably at least 0.6, more preferably 0.7, still more preferably at least 0.8, even more preferably at least 0.9, and most preferably at least 0.95; an odds ratio different from 1, preferably at least about 2 or more or about 0.5 or less, more preferably at least about 3 or more or about 0.33 or less, still more preferably at least about 4 or more or about 0.25 or less, even more preferably at least about 5 or more or about 0.2 or less, and most preferably at least about 10 or more or about 0.1 or less; a positive likelihood ratio (calculated as sensitivity/(1-specificity)) of greater than 1, at least 2, more preferably at least 3, still more preferably at least 5, and most preferably at least 10; and or a negative likelihood ratio (calculated as (1-sensitivity)/specificity) of less than 1, less than or equal to 0.5, more preferably less than or equal to 0.3, and most preferably less than or equal to 0.1

Additional clinical indicia may be combined with the kidney injury marker assay result(s) of the present invention. These include other biomarkers related to renal status. Examples include the following, which recite the common biomarker name, followed by the Swiss-Prot entry number for that biomarker or its parent: Actin (P68133); Adenosine deaminase binding protein (DPP4, P27487); Alpha-1-acid glycoprotein 1 (P02763); Alpha-1-microglobulin (P02760); Albumin (P02768); Angiotensinogenase (Renin, P00797); Annexin A2 (P07355); Beta-glucuronidase (P08236); B-2-microglobulin (P61679); Beta-galactosidase (P16278); BMP-7 (PI8075); Brain natriuretic peptide (proBNP, BNP-32, NTproBNP; P16860); Calcium-binding protein Beta (S100-beta, P04271); Carbonic anhydrase (Q16790); Casein Kinase 2 (P68400); Cadherin-3 (P07858); Ceruloplasmin (P00450); Clusterin (P10909); Complement C3 (P01024); Cysteine-rich protein (CYR61, 000622); Cytochrome C (P99999); Epidermal growth factor (EGF, P01133); Endothelin-1 (P05305); Exosomal Fetuin-A (P02765); Fatty acid-binding protein, heart (FABP3, P05413); Fatty acid-binding protein, liver (P07148); Ferritin (light chain, P02793; heavy chain P02794); Fructose-1,6-biphosphatase (P09467); GRO-alpha (CXCL1, (P09341); Growth Hormone (P01241); Hepatocyte growth factor (P14210); Insulin-like growth factor I (P01343); Immunoglobulin G; Immunoglobulin Light Chains (Kappa and Lambda); Interferon gamma (P01308); Lysozyme (P61626); Interleukin-1alpha (P01583); Interleukin-2 (P60568); Interleukin-4 (P60568); Interleukin-9 (P15248); Interleukin-12p40 (P29460); Interleukin-13 (P35225); Interleukin-16 (Q14005); L1 cell adhesion molecule (P32004); Lactate dehydrogenase (P00338); Leucine Aminopeptidase (P28838); Meprin A-alpha subunit (Q16819); Meprin A-beta subunit (Q16820); Midkine (P21741); MIP2-alpha (CXCL2, P19875); MMP-2 (P08253); MMP-9 (P14780); Netrin-1 (095631); Neutral endopeptidase (P08473); Osteopontin (P10451); Renal papillary antigen 1 (RPA1); Renal papillary antigen 2 (RPA2); Retinol binding protein (P09455); Ribonuclease; S100 calcium-binding protein A6 (P06703); Serum Amyloid P Component (P02743); Sodium/Hydrogen exchanger isoform (NHE3, P48764); Spermidine/spermine N1-acetyltransferase (P21673); TGF-Beta1 (P01137); Transferrin (P02787); Trefoil factor 3 (TFF3, Q07654); Toll-Like protein 4 (000206); Total protein; Tubulointerstitial nephritis antigen (Q9UJW2); Uromodulin (Tamm-Horsfall protein, P07911).

For purposes of risk stratification, Adiponectin (Q15848); Alkaline phosphatase (P05186); Aminopeptidase N (P15144); CalbindinD28k (P05937); Cystatin C (P01034); 8 subunit of FIFO ATPase (P03928); Gamma-glutamyltransferase (P19440); GSTa (alpha-glutathione-S-transferase, P08263); GSTpi (Glutathione-S-transferase P; GST class-pi; P09211); IGFBP-1 (P08833); IGFBP-2 (P18065); IGFBP-6 (P24592); Integral membrane protein 1 (Itm1, P46977); Interleukin-6 (P05231); Interleukin-8 (P10145); Interleukin-18 (Q14116); IP-10 (10 kDa interferon-gamma-induced protein, P02778); IRPR (IFRD1, 000458); Isovaleryl-CoA dehydrogenase (IVD, P26440); I-TAC/CXCL11 (014625); Keratin 19 (P08727); Kim-1 (Hepatitis A virus cellular receptor 1, 043656); L-arginine:glycine amidinotransferase (P50440); Leptin (P41159); Lipocalin2 (NGAL, P80188); MCP-1 (P13500); MIG (Gamma-interferon-induced monokine Q07325); MIP-1a (P10147); MIP-3a (P78556); MIP-1beta (P13236); MIP-1d (Q16663); NAG (N-acetyl-beta-D-glucosaminidase, P54802); Organic ion transporter (OCT2, 015244); Osteoprotegerin (014788); P8 protein (060356); Plasminogen activator inhibitor 1 (PAI-1, P05121); ProANP(1-98) (P01160); Protein phosphatase 1-beta (PPI-beta, P62140); Rab GDI-beta (P50395); Renal kallikrein (Q86U61 ); RT1.B-1 (alpha) chain of the integral membrane protein (Q5Y7A8); Soluble tumor necrosis factor receptor superfamily member 1A (sTNFR-I, P19438); Soluble tumor necrosis factor receptor superfamily member 1B (sTNFR-II, P20333); Tissue inhibitor of metalloproteinases 3 (TIMP-3, P35625); uPAR (Q03405) may be combined with the kidney injury marker assay result(s) of the present invention.

Other clinical indicia which may be combined with the kidney injury marker assay result(s) of the present invention includes demographic information (e.g., weight, sex, age, race), medical history (e.g., family history, type of surgery, pre-existing disease such as aneurism, congestive heart failure, preeclampsia, eclampsia, diabetes mellitus, hypertension, coronary artery disease, proteinuria, renal insufficiency, or sepsis, type of toxin exposure such as NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin), clinical variables (*e.g.*, blood pressure, temperature, respiration rate), risk scores (APACHE score, PREDICT score, TIMI Risk Score for UA/NSTEMI, Framingham Risk Score), a urine total protein measurement, a glomerular filtration rate, an estimated glomerular filtration rate, a urine production rate, a serum or plasma creatinine concentration, a renal papillary antigen 1 (RPA1) measurement; a renal papillary antigen 2 (RPA2) measurement; a urine creatinine concentration, a fractional excretion of sodium, a urine sodium concentration, a urine creatinine to serum or plasma creatinine ratio, a urine specific gravity, a urine osmolality, a urine urea nitrogen to plasma urea nitrogen ratio, a plasma BUN to creatnine ratio, and/or a renal failure index calculated as urine sodium / (urine creatinine / plasma creatinine). Other measures of renal function which may be combined with the kidney injury marker assay result(s) are described hereinafter and in Harrison's Principles of Internal Medicine, 17th Ed., McGraw Hill, New York, pages 1741-1830, and Current Medical Diagnosis & Treatment 2008, 47th Ed, McGraw Hill, New York, pages 785-815.

Combining assay results/clinical indicia in this manner can comprise the use of multivariate logistical regression, loglinear modeling, neural network analysis, n-of-m analysis, decision tree analysis, etc. This list is not meant to be limiting.

Diagnosis of Acute Renal Failure

As noted above, the terms "acute renal (or kidney) injury" and "acute renal (or kidney) failure" as used herein are defined in part in terms of changes in serum creatinine from a baseline value. Most definitions of ARF have common elements, including the use of serum creatinine and, often, urine output. Patients may present with renal dysfunction without an available baseline measure of renal function for use in this comparison. In such an event, one may estimate a baseline serum creatinine value by assuming the patient initially had a normal GFR. Glomerular filtration rate (GFR) is the volume of fluid filtered from the renal (kidney) glomerular capillaries into the Bowman's capsule per unit time. Glomerular filtration rate (GFR) can be calculated by measuring any chemical that has a steady level in the blood, and is freely filtered but neither reabsorbed nor secreted by the kidneys. GFR is typically expressed in units of ml/min:

$$GFR = \frac{\text{Urine Concentration} \times \text{Urine Flow}}{\text{Plasma Concentration}}$$

By normalizing the GFR to the body surface area, a GFR of approximately 75-100 ml/min per 1.73 $m^2$ can be assumed. The rate therefore measured is the quantity of the substance in the urine that originated from a calculable volume of blood.

There are several different techniques used to calculate or estimate the glomerular filtration rate (GFR or eGFR). In clinical practice, however, creatinine clearance is used to measure GFR. Creatinine is produced naturally by the body (creatinine is a metabolite of creatine, which is found in muscle). It is freely filtered by the glomerulus, but also actively secreted by the renal tubules in very small amounts such that creatinine clearance overestimates actual GFR by 10-20%. This margin of error is acceptable considering the ease with which creatinine clearance is measured.

Creatinine clearance (CCr) can be calculated if values for creatinine's urine concentration ($U_{Cr}$), urine flow rate (V), and creatinine's plasma concentration ($P_{Cr}$) are known. Since the product of urine concentration and urine flow rate yields creatinine's excretion rate, creatinine clearance is also said to be its excretion rate ($U_{Cr} \times V$) divided by its plasma concentration. This is commonly represented mathematically as:

$$C_{Cr} = \frac{U_{Cr} \times V}{P_{Cr}}$$

Commonly a 24 hour urine collection is undertaken, from empty-bladder one morning to the contents of the bladder the following morning, with a comparative blood test then taken:

$$C_{Cr} = \frac{U_{Cr} \times 24\text{-hour volume}}{P_{Cr} \times 24 \times 60 mins}$$

To allow comparison of results between people of different sizes, the CCr is often corrected for the body surface area (BSA) and expressed compared to the average sized man as ml/min/1.73 m2. While most adults have a BSA that approaches 1.7 (1.6-1.9), extremely obese or slim patients should have their CCr corrected for their actual BSA:

$$C_{Cr-corrected} = \frac{C_{Cr} \times 1.73}{BSA}$$

The accuracy of a creatinine clearance measurement (even when collection is complete) is limited because as glomerular filtration rate (GFR) falls creatinine secretion is increased, and thus the rise in serum creatinine is less. Thus, creatinine excretion is much greater than the filtered load, resulting in a potentially large overestimation of the GFR (as much as a twofold difference). However, for clinical purposes it is important to determine whether renal function is stable or getting worse or better. This is often determined by monitoring serum creatinine alone. Like creatinine clearance, the serum creatinine will not be an accurate reflection of GFR in the non-steady-state condition of ARF. Nonetheless, the degree to which serum creatinine changes from baseline will reflect the change in GFR. Serum creatinine is readily and easily measured and it is specific for renal function.

For purposes of determining urine output on a Urine output on a mL/kg/hr basis, hourly urine collection and measurement is adequate. In the case where, for example, only a cumulative 24-h output was available and no patient weights are provided, minor modifications of the RIFLE urine output criteria have been described. For example, Bagshaw et al., Nephrol. Dial. Transplant. 23: 1203-1210, 2008, assumes an average patient weight of 70 kg, and patients are assigned a RIFLE classification based on the following: <35 mL/h (Risk), <21 mL/h (Injury) or <4 mL/h (Failure).

Selecting a Treatment Regimen

Once a diagnosis is obtained, the clinician can readily select a treatment regimen that is compatible with the diagnosis, such as initiating renal replacement therapy, withdrawing delivery of compounds that are known to be damaging to the kidney, kidney transplantation, delaying or avoiding procedures that are known to be damaging to the kidney, modifying diuretic administration, initiating goal directed therapy, etc. The skilled artisan is aware of appropriate treatments for numerous diseases discussed in relation to the methods of diagnosis described herein. See, e.g., Merck Manual of Diagnosis and Therapy, 17th Ed. Merck Research Laboratories, Whitehouse Station, NJ, 1999. In addition, since the methods and compositions described herein provide prognostic information, the markers of the present invention may be used to monitor a course of treatment. For example, improved or worsened prognostic state may indicate that a particular treatment is or is not efficacious.

The examples provided herein are representative of preferred embodiments, are exemplary, and are not intended as limitations on the scope of the invention.

Example 1: HA as a diagnostic marker of AKI

Urinary HA and plasma creatinine were measured in mice after administration of folic acid, a known nephrotoxin. Intraperitoneal injections of folic acid (FA, 300mg/kg dissolved in NaHCO$_3$) was selected as a suitable dose to induce AKI (time = 0h) based on pilot studies which indicated that this dose was effective to cause increases in plasma creatinine levels indicative of AKI, but without FA leading to severe illness or death. Control animals received an equivalent volume of vehicle (NaHCO$_3$) i.p. Plasma creatinine and blood urea nitrogen (BUN) were measured to assess renal function using commercially available assays (creatinine kit from Diazyme (San Diego, CA), BUN kit from Sigma (St. Louis, MO)). Urinary HA levels were normalized by expressing the HA concentration per mg of urinary creatinine.

The results of this analysis are depicted in Fig. 1. As can be seen, normalized HA levels are reflective of creatinine levels indicative of AKI in this induced AKI model system.

Example 2: Use of HA as a prognostic and diagnostic marker

Patients from the intensive care unit (ICU) were enrolled in the following study. Each patient was classified by kidney status as non-injury (0), risk of injury (R), injury (I), and failure (F) according to the maximum stage reached within 7 days of enrollment as determined by the RIFLE criteria. EDTA anti-coagulated blood samples (10 mL) and a urine samples (25-30 mL) were collected from each patient at enrollment, 4 ($\pm$ 0.5) and 8 ($\pm$ 1) hours after contrast administration (if applicable); at 12 ($\pm$ 1), 24 ($\pm$ 2), and 48 ($\pm$ 2) hours after enrollment, and thereafter daily up to day 7 to day 14 while the subject is hospitalized. HA was measured by standard immunoassay methods using commercially available assay reagents in the urine samples and the plasma component of the blood samples collected.

Two cohorts were defined as described in the introduction to each of the following tables. In the following tables, the time "prior max stage" represents the time at which a sample is collected, relative to the time a particular patient reaches the lowest disease stage as defined for that cohort, binned into three groups which are +/- 12 hours. For example, "24 hr prior" which uses 0 vs R, I, F as the two cohorts would mean 24 hr (+/- 12 hours) prior to reaching stage R (or I if no sample at R, or F if no sample at R or I).

A receiver operating characteristic (ROC) curve was generated for HA and the area under each ROC curve (AUC) was determined. Patients in Cohort 2 were also separated according to the reason for adjudication to cohort 2 as being based on serum creatinine measurements (sCr), being based on urine output (UO), or being based on either serum creatinine measurements or urine output. Using the same example discussed above (0 vs R, I, F), for those patients adjudicated to stage R, I, or F on the basis of serum creatinine measurements alone, the stage 0 cohort may have included patients adjudicated to stage R, I, or F on the basis of urine output; for those patients adjudicated to stage R, I, or F on the basis of urine output alone, the stage 0 cohort may have included patients adjudicated to stage R, I, or F on the basis of serum creatinine measurements; and for those patients adjudicated to stage R, I, or F on the basis of serum creatinine measurements or urine output, the stage 0 cohort contains only patients in stage 0 for both serum creatinine measurements and urine output. Also, in the data for patients adjudicated on the basis of serum creatinine measurements or urine output, the adjudication method which yielded the most severe RIFLE stage was used.

The ability to distinguish cohort 1 from Cohort 2 was determined using ROC analysis. SE is the standard error of the AUC, n is the number of sample or individual patients ("pts," as indicated). Standard errors were calculated as described in Hanley, J. A., and McNeil, B.J., The meaning and use of the area under a receiver operating characteristic (ROC) curve. Radiology (1982) 143: 29-36; p values were calculated with a two-tailed Z-test. An AUC < 0.5 is indicative of a negative going marker for the comparison, and an AUC > 0.5 is indicative of a positive going marker for the comparison.

Various HA threshold (or "cutoff" concentrations were selected, and the associated sensitivity and specificity for distinguishing cohort 1 from cohort 2 were determined. OR is the odds ratio calculated for the particular cutoff concentration, and 95% CI is the confidence interval for the odds ratio.

Table 1: Comparison of marker levels in urine samples collected from Cohort 1 (patients that did not progress beyond RIFLE stage 0) and in urine samples collected from subjects at 0, 24 hours, and 48 hours prior to reaching stage R, I or F in Cohort 2.

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 979 | 1840 | 979 | 1280 | 979 | 1330 |
| Average | 1290 | 2010 | 1290 | 1870 | 1290 | 2030 |
| Stdev | 1090 | 1300 | 1090 | 1460 | 1090 | 1540 |
| p(t-test) | | 2.3E-13 | | 3.4E-8 | | 3.0E-6 |
| Min | 41.6 | 151 | 41.6 | 77.8 | 41.6 | 126 |
| Max | 6400 | 5710 | 6400 | 6300 | 6400 | 5450 |
| n (Samp) | 570 | 189 | 570 | 170 | 570 | 58 |
| n (Patient) | 259 | 189 | 259 | 170 | 259 | 58 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1280 | 1600 | 1280 | 1550 | 1280 | 1150 |
| Average | 1700 | 1720 | 1700 | 1850 | 1700 | 1750 |
| Stdev | 1350 | 1120 | 1350 | 1290 | 1350 | 1440 |
| p(t-test) | | 0.87 | | 0.39 | | 0.82 |
| Min | 41.6 | 151 | 41.6 | 77.8 | 41.6 | 152 |
| Max | 6400 | 6400 | 6400 | 5710 | 6400 | 5910 |
| n (Samp) | 1322 | 59 | 1322 | 60 | 1322 | 36 |
| n (Patient) | 467 | 59 | 467 | 60 | 467 | 36 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1040 | 2020 | 1040 | 1560 | 1040 | 1500 |
| Average | 1370 | 2230 | 1370 | 2090 | 1370 | 2130 |
| Stdev | 1130 | 1400 | 1130 | 1580 | 1130 | 1550 |
| p(t-test) | | 4.7E-16 | | 1.5E-10 | | 6.0E-6 |
| Min | 41.6 | 168 | 41.6 | 91.1 | 41.6 | 126 |
| Max | 5540 | 6400 | 5540 | 6390 | 5540 | 6190 |
| n (Samp) | 587 | 173 | 587 | 161 | 587 | 54 |
| n (Patient) | 223 | 173 | 223 | 161 | 223 | 54 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.69 | 0.54 | 0.71 | 0.62 | 0.56 | 0.64 | 0.63 | 0.51 | 0.64 |
| SE | 0.024 | 0.039 | 0.024 | 0.025 | 0.039 | 0.026 | 0.041 | 0.049 | 0.042 |
| p | 4.0E-15 | 0.26 | 0 | 1.9E-6 | 0.16 | 8.9E-8 | 9.4E-4 | 0.88 | 5.9E-4 |
| nCohort 1 | 570 | 1322 | 587 | 570 | 1322 | 587 | 570 | 1322 | 587 |
| nCohort 2 | 189 | 59 | 173 | 170 | 60 | 161 | 58 | 36 | 54 |
| Cutoff 1 | 1180 | 1040 | 1360 | 886 | 1100 | 964 | 854 | 849 | 976 |
| Sens 1 | 70% | 71% | 71% | 70% | 70% | 70% | 71% | 72% | 70% |
| Spec 1 | 58% | 40% | 64% | 45% | 42% | 46% | 43% | 32% | 47% |
| Cutoff 2 | 893 | 640 | 1020 | 690 | 770 | 741 | 719 | 648 | 776 |
| Sens 2 | 80% | 81% | 80% | 80% | 80% | 80% | 81% | 81% | 81% |
| Spec 2 | 46% | 22% | 49% | 35% | 29% | 35% | 37% | 22% | 38% |
| Cutoff 3 | 451 | 358 | 583 | 392 | 389 | 465 | 437 | 477 | 437 |
| Sens 3 | 90% | 92% | 90% | 90% | 90% | 90% | 91% | 92% | 91% |
| Spec 3 | 19% | 9% | 25% | 16% | 10% | 18% | 19% | 15% | 17% |
| Cutoff 4 | 1480 | 2010 | 1600 | 1480 | 2010 | 1600 | 1480 | 2010 | 1600 |
| Sens 4 | 61% | 37% | 65% | 46% | 37% | 49% | 47% | 36% | 48% |
| Spec 4 | 70% | 70% | 70% | 70% | 70% | 70% | 70% | 70% | 70% |
| Cutoff 5 | 1820 | 2610 | 2010 | 1820 | 2610 | 2010 | 1820 | 2610 | 2010 |
| Sens 5 | 52% | 19% | 50% | 42% | 20% | 42% | 45% | 19% | 46% |
| Spec 5 | 80% | 80% | 80% | 80% | 80% | 80% | 80% | 80% | 80% |
| Cutoff 6 | 2660 | 3790 | 2890 | 2660 | 3790 | 2890 | 2660 | 3790 | 2890 |
| Sens 6 | 25% | 2% | 24% | 25% | 8% | 27% | 34% | 11% | 33% |
| Spec 6 | 90% | 90% | 90% | 90% | 90% | 90% | 90% | 90% | 90% |
| OR Quart 2 | 1.1 | 0.66 | 1.3 | 1.6 | 1.6 | 2.6 | 1.8 | 1.1 | 1.3 |
| p Value | 0.79 | 0.37 | 0.43 | 0.10 | 0.24 | 0.0013 | 0.19 | 0.82 | 0.63 |
| 95% CI of | 0.61 | 0.27 | 0.69 | 0.91 | 0.73 | 1.4 | 0.74 | 0.45 | 0.49 |
| OR Quart2 | 1.9 | 1.6 | 2.4 | 2.7 | 3.6 | 4.6 | 4.5 | 2.8 | 3.3 |
| OR Quart 3 | 1.9 | 1.8 | 2.7 | 1.4 | 1.6 | 1.5 | 1.3 | 0.89 | 1.4 |
| p Value | 0.015 | 0.11 | 5.9E-4 | 0.21 | 0.23 | 0.22 | 0.63 | 0.81 | 0.48 |
| 95% CI of | 1.1 | 0.87 | 1.5 | 0.82 | 0.73 | 0.80 | 0.49 | 0.34 | 0.55 |
| OR Quart3 | 3.2 | 3.7 | 4.8 | 2.5 | 3.6 | 2.7 | 3.3 | 2.3 | 3.6 |
| OR Quart 4 | 5.1 | 1.5 | 6.5 | 3.8 | 1.8 | 4.9 | 3.7 | 1.00 | 3.5 |
| p Value | 1.6E-10 | 0.27 | 2.0E-11 | 2.7E-7 | 0.13 | 1.7E-8 | 0.0019 | 0.99 | 0.0031 |
| 95% CI of | 3.1 | 0.72 | 3.7 | 2.3 | 0.84 | 2.8 | 1.6 | 0.39 | 1.5 |
| OR Quart4 | 8.3 | 3.2 | 11 | 6.3 | 4.0 | 8.5 | 8.4 | 2.5 | 8.0 |

Table 2: Comparison of marker levels in urine samples collected from Cohort 1 (patients that did not progress beyond RIFLE stage 0 or R) and in urine samples collected from subjects at 0, 24 hours, and 48 hours prior to reaching stage I or F in Cohort 2.

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1180 | 2190 | 1180 | 2050 | 1180 | 1880 |
| Average | 1500 | 2440 | 1500 | 2450 | 1500 | 2100 |
| Stdev | 1190 | 1460 | 1190 | 1650 | 1190 | 1620 |
| p(t-test) | | 1.3E-13 | | 7.7E-14 | | 2.0E-4 |
| Min | 41.6 | 89.4 | 41.6 | 110 | 41.6 | 81.2 |
| Max | 6400 | 6400 | 6400 | 6400 | 6400 | 6190 |
| n (Samp) | 1183 | 102 | 1183 | 106 | 1183 | 61 |
| n (Patient) | 444 | 102 | 444 | 106 | 444 | 61 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1330 | 1760 | 1330 | 2010 | 1330 | 1550 |
| Average | 1740 | 2060 | 1740 | 2430 | 1740 | 1970 |
| Stdev | 1380 | 1260 | 1380 | 1470 | 1380 | 1530 |
| p(t-test) | | 0.28 | | 0.0085 | | 0.42 |
| Min | 41.6 | 404 | 41.6 | 340 | 41.6 | 324 |
| Max | 6400 | 6400 | 6400 | 6400 | 6400 | 6400 |
| n (Samp) | 1617 | 22 | 1617 | 29 | 1617 | 25 |
| n (Patient) | 556 | 22 | 556 | 29 | 556 | 25 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1220 | 2330 | 1220 | 2180 | 1220 | 1950 |
| Average | 1550 | 2600 | 1550 | 2510 | 1550 | 2290 |
| Stdev | 1200 | 1530 | 1200 | 1700 | 1200 | 1700 |
| p(t-test) | | 5.8E-15 | | 5.4E-13 | | 2.3E-5 |
| Min | 41.6 | 89.4 | 41.6 | 110 | 41.6 | 81.2 |
| Max | 6400 | 6400 | 6400 | 6400 | 6400 | 6190 |
| n (Samp) | 1118 | 93 | 1118 | 97 | 1118 | 52 |
| n (Patient) | 382 | 93 | 382 | 97 | 382 | 52 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.71 | 0.62 | 0.71 | 0.68 | 0.66 | 0.67 | 0.60 | 0.55 | 0.62 |
| SE | 0.030 | 0.064 | 0.031 | 0.030 | 0.056 | 0.031 | 0.039 | 0.060 | 0.042 |
| p | 2.0E-12 | 0.069 | 3.6E-12 | 1.5E-9 | 0.0033 | 2.0E-8 | 0.012 | 0.36 | 0.0051 |
| nCohort 1 | 1183 | 1617 | 1118 | 1183 | 1617 | 1118 | 1183 | 1617 | 1118 |
| nCohort 2 | 102 | 22 | 93 | 106 | 29 | 97 | 61 | 25 | 52 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 1 | 1590 | 1340 | 1660 | 1330 | 1710 | 1400 | 886 | 1100 | 957 |
| Sens 1 | 71% | 73% | 71% | 71% | 72% | 70% | 70% | 72% | 71% |
| Spec 1 | 65% | 50% | 65% | 57% | 61% | 57% | 37% | 41% | 38% |
| Cutoff 2 | 1160 | 1070 | 1190 | 923 | 1150 | 819 | 582 | 770 | 648 |
| Sens 2 | 80% | 82% | 81% | 80% | 83% | 80% | 80% | 80% | 81% |
| Spec 2 | 49% | 40% | 49% | 39% | 42% | 32% | 22% | 28% | 23% |
| Cutoff 3 | 671 | 1020 | 671 | 515 | 641 | 513 | 469 | 537 | 470 |
| Sens 3 | 90% | 91% | 90% | 91% | 93% | 91% | 90% | 92% | 90% |
| Spec 3 | 26% | 38% | 25% | 18% | 21% | 17% | 16% | 17% | 15% |
| Cutoff 4 | 1770 | 2050 | 1850 | 1770 | 2050 | 1850 | 1770 | 2050 | 1850 |
| Sens 4 | 62% | 41% | 66% | 58% | 48% | 59% | 51% | 36% | 54% |
| Spec 4 | 70% | 70% | 70% | 70% | 70% | 70% | 70% | 70% | 70% |
| Cutoff 5 | 2160 | 2700 | 2280 | 2160 | 2700 | 2280 | 2160 | 2700 | 2280 |
| Sens 5 | 51% | 18% | 51% | 48% | 31% | 47% | 39% | 16% | 40% |
| Spec 5 | 80% | 80% | 80% | 80% | 80% | 80% | 80% | 80% | 80% |
| Cutoff 6 | 3280 | 3830 | 3390 | 3280 | 3830 | 3390 | 3280 | 3830 | 3390 |
| Sens 6 | 24% | 5% | 27% | 24% | 14% | 26% | 26% | 12% | 29% |
| Spec 6 | 90% | 90% | 90% | 90% | 90% | 90% | 90% | 90% | 90% |
| OR Quart 2 | 1.3 | 5.0 | 1.2 | 1.1 | 1.00 | 1.2 | 0.59 | 1.2 | 0.49 |
| p Value | 0.53 | 0.14 | 0.67 | 0.85 | 1.00 | 0.71 | 0.22 | 0.76 | 0.16 |
| 95% CI of OR Quart2 | 0.57 | 0.59 | 0.51 | 0.51 | 0.20 | 0.54 | 0.25 | 0.36 | 0.18 |
| | 3.0 | 43 | 2.8 | 2.3 | 5.0 | 2.5 | 1.4 | 4.0 | 1.3 |
| OR Quart 3 | 2.5 | 8.1 | 1.8 | 1.8 | 3.7 | 1.5 | 0.59 | 1.6 | 0.91 |
| p Value | 0.017 | 0.049 | 0.13 | 0.098 | 0.044 | 0.28 | 0.22 | 0.41 | 0.83 |
| 95% CI of OR Quart3 | 1.2 | 1.0 | 0.84 | 0.90 | 1.0 | 0.72 | 0.25 | 0.52 | 0.40 |
| | 5.3 | 65 | 4.1 | 3.5 | 14 | 3.1 | 1.4 | 5.0 | 2.1 |
| OR Quart 4 | 6.4 | 8.1 | 6.2 | 4.3 | 4.1 | 4.4 | 2.0 | 1.2 | 2.0 |
| p Value | 1.5E-7 | 0.049 | 2.9E-7 | 2.7E-6 | 0.030 | 4.6E-6 | 0.043 | 0.76 | 0.061 |
| 95% CI of OR Quart4 | 3.2 | 1.0 | 3.1 | 2.3 | 1.1 | 2.3 | 1.0 | 0.36 | 0.97 |
| | 13 | 65 | 12 | 8.0 | 15 | 8.3 | 3.7 | 4.0 | 4.1 |

Table 3: Comparison of marker levels in urine samples collected within 12 hours of reaching stage R from Cohort 1 (patients that reached, but did not progress beyond, RIFLE stage R) and from Cohort 2 (patients that reached RIFLE stage I or F).

|            | sCr or UO |          | sCr only |          | UO only  |          |
|------------|-----------|----------|----------|----------|----------|----------|
|            | Cohort 1  | Cohort 2 | Cohort 1 | Cohort 2 | Cohort 1 | Cohort 2 |
| Median     | 1680      | 2050     | 1600     | 2430     | 1850     | 1870     |
| Average    | 1830      | 2300     | 1950     | 2470     | 1950     | 2220     |
| Stdev      | 1160      | 1540     | 1410     | 1360     | 1130     | 1550     |
| p(t-test)  |           | 0.0071   |          | 0.15     |          | 0.16     |
| Min        | 151       | 183      | 151      | 183      | 168      | 190      |
| Max        | 5180      | 6350     | 6400     | 5250     | 5180     | 6400     |
| n (Samp)   | 169       | 84       | 65       | 20       | 142      | 64       |
| n (Patient)| 169       | 84       | 65       | 20       | 142      | 64       |

|  | At Enrollment | | |
|---|---|---|---|
|  | sCr or UO | sCr only | UO only |
| AUC | 0.58 | 0.62 | 0.53 |
| SE | 0.039 | 0.075 | 0.044 |
| p | 0.033 | 0.097 | 0.55 |
| nCohort 1 | 169 | 65 | 142 |
| nCohort 2 | 84 | 20 | 64 |
| Cutoff 1 | 1270 | 1940 | 1270 |
| Sens 1 | 70% | 70% | 70% |
| Spec 1 | 36% | 60% | 31% |
| Cutoff 2 | 945 | 1400 | 1000 |
| Sens 2 | 81% | 80% | 81% |
| Spec 2 | 23% | 38% | 22% |
| Cutoff 3 | 550 | 842 | 582 |
| Sens 3 | 90% | 90% | 91% |
| Spec 3 | 13% | 26% | 10% |
| Cutoff 4 | 2150 | 2560 | 2280 |
| Sens 4 | 48% | 50% | 34% |
| Spec 4 | 70% | 71% | 70% |
| Cutoff 5 | 2700 | 2940 | 2770 |
| Sens 5 | 32% | 35% | 23% |
| Spec 5 | 80% | 80% | 80% |
| Cutoff 6 | 3530 | 3790 | 3470 |
| Sens 6 | 18% | 15% | 17% |
| Spec 6 | 91% | 91% | 90% |
| OR Quart 2 | 0.93 | 0.63 | 1.4 |
| p Value | 0.84 | 0.64 | 0.43 |
| 95% CI of | 0.43 | 0.094 | 0.60 |
| OR Quart2 | 2.0 | 4.2 | 3.2 |
| OR Quart 3 | 1.0 | 3.7 | 1.1 |
| p Value | 1.0 | 0.089 | 0.83 |
| 95% CI of | 0.47 | 0.82 | 0.47 |
| OR Quart3 | 2.1 | 17 | 2.6 |
| OR Quart 4 | 1.8 | 2.8 | 1.3 |
| p Value | 0.11 | 0.18 | 0.56 |
| 95% CI of | 0.87 | 0.61 | 0.55 |
| OR Quart4 | 3.7 | 13 | 3.0 |

Table 4: Comparison of the maximum marker levels in urine samples collected from Cohort 1 (patients that did not progress beyond RIFLE stage 0) and the maximum values in urine samples collected from subjects between enrollment and 0, 24 hours, and 48 hours prior to reaching stage F in Cohort 2.

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1250 | 3410 | 1250 | 3300 | 1250 | 3210 |
| Average | 1570 | 3520 | 1570 | 3470 | 1570 | 3050 |
| Stdev | 1190 | 1570 | 1190 | 1580 | 1190 | 1230 |
| p(t-test) | | 3.9E-19 | | 5.2E-18 | | 1.6E-8 |
| Min | 69.2 | 565 | 69.2 | 565 | 69.2 | 1020 |
| Max | 6400 | 6400 | 6400 | 6400 | 6400 | 6190 |
| n (Samp) | 259 | 44 | 259 | 43 | 259 | 24 |
| n (Patient) | 259 | 44 | 259 | 43 | 259 | 24 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1760 | 3240 | 1760 | 3170 | 1760 | 3110 |
| Average | 2100 | 3070 | 2100 | 2930 | 2100 | 2860 |
| Stdev | 1470 | 1530 | 1470 | 1380 | 1470 | 945 |
| p(t-test) | | 0.0033 | | 0.012 | | 0.056 |
| Min | 69.2 | 565 | 69.2 | 565 | 69.2 | 1330 |
| Max | 6400 | 6400 | 6400 | 5080 | 6400 | 4360 |
| n (Samp) | 467 | 21 | 467 | 21 | 467 | 14 |
| n (Patient) | 467 | 21 | 467 | 21 | 467 | 14 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1400 | 3720 | 1400 | 3600 | 1400 | 3130 |
| Average | 1790 | 3850 | 1790 | 3800 | 1790 | 3090 |
| Stdev | 1250 | 1590 | 1250 | 1610 | 1250 | 1400 |
| p(t-test) | | 3.0E-15 | | 2.8E-14 | | 3.7E-5 |
| Min | 113 | 687 | 113 | 687 | 113 | 1020 |
| Max | 5540 | 6400 | 5540 | 6400 | 5540 | 6190 |
| n (Samp) | 223 | 32 | 223 | 31 | 223 | 18 |
| n (Patient) | 223 | 32 | 223 | 31 | 223 | 18 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.84 | 0.69 | 0.85 | 0.83 | 0.68 | 0.84 | 0.83 | 0.71 | 0.78 |
| SE | 0.039 | 0.065 | 0.044 | 0.040 | 0.066 | 0.045 | 0.053 | 0.079 | 0.066 |
| p | 0 | 0.0035 | 5.6E-15 | 0 | 0.0064 | 7.5E-14 | 3.5E-10 | 0.0095 | 2.3E-5 |
| nCohort 1 | 259 | 467 | 223 | 259 | 467 | 223 | 259 | 467 | 223 |
| nCohort 2 | 44 | 21 | 32 | 43 | 21 | 31 | 24 | 14 | 18 |
| Cutoff 1 | 2720 | 2210 | 2910 | 2710 | 2210 | 2800 | 2200 | 2210 | 2170 |
| Sens 1 | 70% | 71% | 72% | 72% | 71% | 71% | 71% | 71% | 72% |
| Spec 1 | 85% | 65% | 84% | 85% | 65% | 83% | 79% | 65% | 73% |
| Cutoff 2 | 2170 | 1810 | 2470 | 2170 | 1810 | 2470 | 1810 | 1810 | 1660 |
| Sens 2 | 82% | 81% | 81% | 81% | 81% | 81% | 83% | 86% | 83% |
| Spec 2 | 79% | 52% | 77% | 79% | 52% | 77% | 70% | 52% | 58% |
| Cutoff 3 | 1060 | 1060 | 1810 | 1060 | 1060 | 1810 | 1590 | 1590 | 1320 |
| Sens 3 | 91% | 90% | 91% | 91% | 90% | 90% | 92% | 93% | 94% |
| Spec 3 | 42% | 28% | 63% | 42% | 28% | 63% | 63% | 45% | 48% |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 4 | 1860 | 2450 | 2120 | 1860 | 2450 | 2120 | 1860 | 2450 | 2120 |
| Sens 4 | 84% | 67% | 88% | 84% | 67% | 87% | 79% | 64% | 72% |
| Spec 4 | 70% | 70% | 70% | 70% | 70% | 70% | 70% | 70% | 70% |
| Cutoff 5 | 2270 | 3280 | 2630 | 2270 | 3280 | 2630 | 2270 | 3280 | 2630 |
| Sens 5 | 77% | 43% | 78% | 77% | 38% | 77% | 67% | 36% | 67% |
| Spec 5 | 80% | 80% | 80% | 80% | 80% | 80% | 80% | 80% | 80% |
| Cutoff 6 | 3260 | 4350 | 3660 | 3260 | 4350 | 3660 | 3260 | 4350 | 3660 |
| Sens 6 | 57% | 19% | 50% | 53% | 19% | 48% | 46% | 7% | 22% |
| Spec 6 | 90% | 90% | 90% | 90% | 90% | 90% | 90% | 90% | 90% |
| OR Quart 2 | 2.0 | 1.0 | 2.0 | 0.99 | 1.0 | 2.0 | >1.0 | >2.0 | >2.1 |
| p Value | 0.42 | 1.0 | 0.58 | 0.99 | 1.0 | 0.58 | <1.0 | <0.56 | <0.56 |
| 95% CI of | 0.36 | 0.14 | 0.18 | 0.19 | 0.14 | 0.18 | >0.061 | >0.18 | >0.18 |
| OR Quart2 | 11 | 7.2 | 23 | 5.0 | 7.2 | 23 | na | na | na |
| OR Quart 3 | 3.1 | 2.6 | 5.3 | 2.1 | 2.6 | 4.2 | >7.7 | >4.1 | >4.3 |
| p Value | 0.17 | 0.27 | 0.14 | 0.31 | 0.27 | 0.20 | <0.060 | <0.21 | <0.20 |
| 95% CI of | 0.61 | 0.49 | 0.60 | 0.50 | 0.49 | 0.46 | >0.92 | >0.46 | >0.46 |
| OR Quart3 | 16 | 13 | 46 | 8.7 | 13 | 39 | na | na | na |
| OR Quart 4 | 27 | 6.5 | 37 | 17 | 6.5 | 37 | >20 | >8.5 | >15 |
| p Value | 1.4E-5 | 0.015 | 5.1E-4 | 9.6E-6 | 0.015 | 5.1E-4 | <0.0040 | <0.045 | <0.011 |
| 95% CI of | 6.1 | 1.4 | 4.8 | 4.8 | 1.4 | 4.8 | >2.6 | >1.0 | >1.8 |
| OR Quart4 | 120 | 30 | 290 | 57 | 30 | 290 | na | na | na |

Table 5: Comparison of marker levels in EDTA samples collected from Cohort 1 (patients that did not progress beyond RIFLE stage 0) and in EDTA samples collected from subjects at 0, 24 hours, and 48 hours prior to reaching stage R, I or F in Cohort 2.

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 284 | 335 | 284 | 331 | 284 | 428 |
| Average | 501 | 570 | 501 | 708 | 501 | 930 |
| Stdev | 627 | 641 | 627 | 839 | 627 | 999 |
| p(t-test) | | 0.43 | | 0.053 | | 0.021 |
| Min | 86.8 | 74.7 | 86.8 | 63.6 | 86.8 | 132 |
| Max | 3370 | 3170 | 3370 | 3200 | 3370 | 3200 |
| n (Samp) | 162 | 77 | 162 | 56 | 162 | 14 |
| n (Patient) | 90 | 77 | 90 | 56 | 90 | 14 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 290 | 350 | 290 | 573 | 290 | 309 |
| Average | 619 | 505 | 619 | 540 | 619 | 374 |
| Stdev | 764 | 488 | 764 | 251 | 764 | 269 |
| p(t-test) | | 0.50 | | 0.71 | | 0.43 |
| Min | 48.0 | 105 | 48.0 | 183 | 48.0 | 112 |
| Max | 3370 | 2060 | 3370 | 1020 | 3370 | 832 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| n (Samp) | 378 | 21 | 378 | 13 | 378 | 6 |
| n (Patient) | 178 | 21 | 178 | 13 | 178 | 6 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 323 | 384 | 323 | 330 | 323 | 499 |
| Average | 544 | 626 | 544 | 724 | 544 | 1070 |
| Stdev | 603 | 688 | 603 | 863 | 603 | 1080 |
| p(t-test) | | 0.36 | | 0.075 | | 0.0012 |
| Min | 86.8 | 74.7 | 86.8 | 63.6 | 86.8 | 132 |
| Max | 3370 | 3170 | 3370 | 3200 | 3370 | 3200 |
| n (Samp) | 187 | 66 | 187 | 59 | 187 | 18 |
| n (Patient) | 94 | 66 | 94 | 59 | 94 | 18 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.56 | 0.52 | 0.54 | 0.57 | 0.65 | 0.53 | 0.65 | 0.45 | 0.65 |
| SE | 0.040 | 0.066 | 0.042 | 0.045 | 0.084 | 0.044 | 0.082 | 0.12 | 0.073 |
| p | 0.16 | 0.73 | 0.33 | 0.12 | 0.077 | 0.56 | 0.070 | 0.70 | 0.039 |
| nCohort 1 | 162 | 378 | 187 | 162 | 378 | 187 | 162 | 378 | 187 |
| nCohort 2 | 77 | 21 | 66 | 56 | 13 | 59 | 14 | 6 | 18 |
| Cutoff 1 | 246 | 280 | 248 | 217 | 326 | 217 | 317 | 156 | 317 |
| Sens 1 | 70% | 71% | 71% | 71% | 77% | 71% | 71% | 83% | 72% |
| Spec 1 | 40% | 47% | 36% | 33% | 55% | 28% | 55% | 15% | 49% |
| Cutoff 2 | 198 | 194 | 217 | 190 | 318 | 190 | 182 | 156 | 212 |
| Sens 2 | 81% | 81% | 80% | 80% | 85% | 81% | 86% | 83% | 83% |
| Spec 2 | 28% | 25% | 28% | 27% | 53% | 22% | 26% | 15% | 27% |
| Cutoff 3 | 124 | 124 | 141 | 150 | 232 | 141 | 168 | 111 | 168 |
| Sens 3 | 91% | 90% | 91% | 91% | 92% | 92% | 93% | 100% | 94% |
| Spec 3 | 10% | 8% | 12% | 17% | 35% | 12% | 23% | 4% | 19% |
| Cutoff 4 | 409 | 491 | 501 | 409 | 491 | 501 | 409 | 491 | 501 |
| Sens 4 | 40% | 33% | 29% | 48% | 54% | 36% | 50% | 33% | 50% |
| Spec 4 | 70% | 70% | 70% | 70% | 70% | 70% | 70% | 70% | 70% |
| Cutoff 5 | 578 | 833 | 751 | 578 | 833 | 751 | 578 | 833 | 751 |
| Sens 5 | 25% | 14% | 26% | 32% | 15% | 24% | 43% | 0% | 44% |
| Spec 5 | 80% | 80% | 80% | 80% | 80% | 80% | 80% | 80% | 80% |
| Cutoff 6 | 1030 | 1820 | 1320 | 1030 | 1820 | 1320 | 1030 | 1820 | 1320 |
| Sens 6 | 12% | 5% | 12% | 20% | 0% | 20% | 29% | 0% | 28% |
| Spec 6 | 90% | 90% | 90% | 90% | 90% | 90% | 90% | 90% | 90% |
| OR Quart 2 | 1.4 | 0.73 | 1.4 | 1.5 | 0.99 | 0.89 | 1.0 | 2.0 | 1.0 |
| p Value | 0.45 | 0.69 | 0.40 | 0.40 | 0.99 | 0.79 | 1.0 | 0.57 | 1.0 |
| 95% CI of OR Quart2 | 0.61 | 0.16 | 0.62 | 0.60 | 0.061 | 0.39 | 0.13 | 0.18 | 0.19 |
| | 3.0 | 3.4 | 3.2 | 3.6 | 16 | 2.1 | 7.4 | 23 | 5.2 |
| OR Quart 3 | 1.6 | 2.3 | 1.5 | 1.0 | 5.2 | 0.83 | 2.1 | 1.0 | 1.4 |
| p Value | 0.26 | 0.17 | 0.30 | 1.0 | 0.14 | 0.67 | 0.41 | 1.0 | 0.70 |
| 95% CI of OR Quart3 | 0.72 | 0.70 | 0.68 | 0.39 | 0.59 | 0.36 | 0.36 | 0.062 | 0.29 |
| | 3.5 | 7.9 | 3.5 | 2.6 | 45 | 1.9 | 12 | 16 | 6.4 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| OR Quart 4 | 1.7 | 1.2 | 1.5 | 2.1 | 6.3 | 1.2 | 3.3 | 2.0 | 2.9 |
| p Value | 0.19 | 0.75 | 0.33 | 0.10 | 0.092 | 0.72 | 0.16 | 0.57 | 0.13 |
| 95% CI of | 0.77 | 0.33 | 0.66 | 0.87 | 0.74 | 0.52 | 0.63 | 0.18 | 0.73 |
| OR Quart4 | 3.7 | 4.8 | 3.4 | 4.9 | 53 | 2.6 | 17 | 23 | 12 |

Table 6: Comparison of marker levels in EDTA samples collected from Cohort 1 (patients that did not progress beyond RIFLE stage 0 or R) and in EDTA samples collected from subjects at 0, 24 hours, and 48 hours prior to reaching stage I or F in Cohort 2.

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 317 | 318 | 317 | 318 | 317 | 524 |
| Average | 581 | 651 | 581 | 739 | 581 | 729 |
| Stdev | 680 | 806 | 680 | 882 | 680 | 794 |
| p(t-test) | | 0.61 | | 0.19 | | 0.36 |
| Min | 74.7 | 113 | 74.7 | 48.0 | 74.7 | 112 |
| Max | 3370 | 2880 | 3370 | 3200 | 3370 | 2810 |
| n (Samp) | 357 | 28 | 357 | 37 | 357 | 19 |
| n (Patient) | 179 | 28 | 179 | 37 | 179 | 19 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | nd | nd | 333 | 469 |
| Average | nd | nd | nd | nd | 647 | 452 |
| Stdev | nd | nd | nd | nd | 751 | 285 |
| p(t-test) | nd | nd | nd | nd | | 0.53 |
| Min | nd | nd | nd | nd | 48.0 | 112 |
| Max | nd | nd | nd | nd | 3370 | 832 |
| n (Samp) | nd | nd | nd | nd | 477 | 6 |
| n (Patient) | nd | nd | nd | nd | 216 | 6 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 325 | 303 | 325 | 314 | 325 | 524 |
| Average | 586 | 638 | 586 | 745 | 586 | 760 |
| Stdev | 671 | 810 | 671 | 893 | 671 | 827 |
| p(t-test) | | 0.70 | | 0.19 | | 0.30 |
| Min | 74.7 | 113 | 74.7 | 48.0 | 74.7 | 119 |
| Max | 3370 | 2880 | 3370 | 3200 | 3370 | 2810 |
| n (Samp) | 347 | 28 | 347 | 36 | 347 | 17 |
| n (Patient) | 167 | 28 | 167 | 36 | 167 | 17 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.52 | nd | 0.49 | 0.54 | nd | 0.52 | 0.54 | 0.50 | 0.55 |
| SE | 0.057 | nd | 0.057 | 0.051 | nd | 0.051 | 0.069 | 0.12 | 0.073 |
| p | 0.76 | nd | 0.89 | 0.48 | nd | 0.67 | 0.54 | 0.97 | 0.53 |
| nCohort 1 | 357 | nd | 347 | 357 | nd | 347 | 357 | 477 | 347 |
| nCohort 2 | 28 | nd | 28 | 37 | nd | 36 | 19 | 6 | 17 |
| Cutoff 1 | 246 | nd | 246 | 228 | nd | 227 | 184 | 194 | 194 |
| Sens 1 | 71% | nd | 71% | 70% | nd | 72% | 74% | 83% | 71% |
| Spec 1 | 37% | nd | 35% | 33% | nd | 31% | 22% | 23% | 22% |
| Cutoff 2 | 168 | nd | 168 | 191 | nd | 191 | 141 | 194 | 183 |
| Sens 2 | 82% | nd | 82% | 81% | nd | 81% | 84% | 83% | 82% |
| Spec 2 | 19% | nd | 17% | 24% | nd | 22% | 13% | 23% | 20% |
| Cutoff 3 | 141 | nd | 141 | 112 | nd | 111 | 118 | 111 | 128 |
| Sens 3 | 93% | nd | 93% | 92% | nd | 92% | 95% | 100% | 94% |
| Spec 3 | 13% | nd | 11% | 4% | nd | 3% | 6% | 4% | 8% |
| Cutoff 4 | 502 | nd | 512 | 502 | nd | 512 | 502 | 535 | 512 |
| Sens 4 | 29% | nd | 25% | 41% | nd | 42% | 53% | 50% | 53% |
| Spec 4 | 70% | nd | 70% | 70% | nd | 70% | 70% | 70% | 70% |
| Cutoff 5 | 833 | nd | 841 | 833 | nd | 841 | 833 | 940 | 841 |
| Sens 5 | 18% | nd | 18% | 24% | nd | 25% | 21% | 0% | 24% |
| Spec 5 | 80% | nd | 80% | 80% | nd | 80% | 80% | 80% | 80% |
| Cutoff 6 | 1410 | nd | 1400 | 1410 | nd | 1400 | 1410 | 1860 | 1400 |
| Sens 6 | 14% | nd | 14% | 19% | nd | 19% | 16% | 0% | 18% |
| Spec 6 | 90% | nd | 90% | 90% | nd | 90% | 90% | 90% | 90% |
| OR Quart 2 | 1.0 | nd | 1.2 | 0.99 | nd | 1.2 | 0.32 | 2.0 | 0.32 |
| p Value | 1.0 | nd | 0.77 | 0.98 | nd | 0.65 | 0.17 | 0.57 | 0.17 |
| 95% CI of | 0.34 | nd | 0.38 | 0.38 | nd | 0.49 | 0.063 | 0.18 | 0.063 |
| OR Quart2 | 3.0 | nd | 3.7 | 2.6 | nd | 3.1 | 1.6 | 23 | 1.6 |
| OR Quart 3 | 1.0 | nd | 1.4 | 0.88 | nd | 0.64 | 0.65 | 1.0 | 0.65 |
| p Value | 1.0 | nd | 0.58 | 0.80 | nd | 0.41 | 0.52 | 1.0 | 0.52 |
| 95% CI of | 0.34 | nd | 0.45 | 0.32 | nd | 0.22 | 0.18 | 0.062 | 0.18 |
| OR Quart3 | 3.0 | nd | 4.1 | 2.4 | nd | 1.9 | 2.4 | 16 | 2.4 |
| OR Quart 4 | 0.99 | nd | 1.2 | 1.2 | nd | 1.1 | 1.2 | 2.0 | 0.82 |
| p Value | 0.98 | nd | 0.76 | 0.65 | nd | 0.83 | 0.77 | 0.56 | 0.76 |
| 95% CI of | 0.33 | nd | 0.39 | 0.49 | nd | 0.43 | 0.38 | 0.18 | 0.24 |
| OR Quart4 | 2.9 | nd | 3.7 | 3.1 | nd | 2.9 | 3.7 | 23 | 2.8 |

Table 7: Comparison of marker levels in EDTA samples collected within 12 hours of reaching stage R from Cohort 1 (patients that reached, but did not progress beyond, RIFLE stage R) and from Cohort 2 (patients that reached RIFLE stage I or F).

|          | sCr or UO |          | sCr only |          | UO only  |          |
|----------|-----------|----------|----------|----------|----------|----------|
|          | Cohort 1  | Cohort 2 | Cohort 1 | Cohort 2 | Cohort 1 | Cohort 2 |
| Median   | 316       | 336      | nd       | nd       | 335      | 348      |
| Average  | 608       | 776      | nd       | nd       | 591      | 728      |
| Stdev    | 666       | 936      | nd       | nd       | 664      | 883      |

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| p(t-test) | | 0.32 | nd | nd | | 0.45 |
| Min | 74.7 | 110 | nd | nd | 74.7 | 110 |
| Max | 3200 | 3170 | nd | nd | 3200 | 3170 |
| n (Samp) | 67 | 30 | nd | nd | 51 | 26 |
| n (Patient) | 67 | 30 | nd | nd | 51 | 26 |

| | At Enrollment | | |
|---|---|---|---|
| | sCr or UO | sCr only | UO only |
| AUC | 0.53 | nd | 0.52 |
| SE | 0.064 | nd | 0.070 |
| p | 0.65 | nd | 0.75 |
| nCohort 1 | 67 | nd | 51 |
| nCohort 2 | 30 | nd | 26 |
| Cutoff 1 | 262 | nd | 219 |
| Sens 1 | 70% | nd | 73% |
| Spec 1 | 39% | nd | 27% |
| Cutoff 2 | 194 | nd | 186 |
| Sens 2 | 80% | nd | 81% |
| Spec 2 | 19% | nd | 18% |
| Cutoff 3 | 173 | nd | 159 |
| Sens 3 | 90% | nd | 92% |
| Spec 3 | 19% | nd | 18% |
| Cutoff 4 | 685 | nd | 538 |
| Sens 4 | 27% | nd | 27% |
| Spec 4 | 70% | nd | 71% |
| Cutoff 5 | 900 | nd | 849 |
| Sens 5 | 27% | nd | 23% |
| Spec 5 | 81% | nd | 80% |
| Cutoff 6 | 1410 | nd | 1200 |
| Sens 6 | 20% | nd | 19% |
| Spec 6 | 91% | nd | 90% |
| OR Quart 2 | 1.0 | nd | 1.3 |
| p Value | 1.0 | nd | 0.73 |
| 95% CI of | 0.29 | nd | 0.33 |
| OR Quart2 | 3.5 | nd | 4.8 |
| OR Quart 3 | 1.2 | nd | 1.3 |
| p Value | 0.76 | nd | 0.73 |
| 95% CI of | 0.36 | nd | 0.33 |
| OR Quart3 | 4.1 | nd | 4.8 |
| OR Quart 4 | 1.1 | nd | 0.93 |
| p Value | 0.83 | nd | 0.91 |
| 95% CI of | 0.34 | nd | 0.24 |
| OR Quart4 | 3.9 | nd | 3.6 |

Table 8: Comparison of the maximum marker levels in EDTA samples collected from Cohort 1 (patients that did not progress beyond RIFLE stage 0) and

the maximum values in EDTA samples collected from subjects between enrollment and 0, 24 hours, and 48 hours prior to reaching stage F in Cohort 2.

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 345 | 698 | 345 | 698 | 345 | 663 |
| Average | 612 | 1270 | 612 | 1090 | 612 | 578 |
| Stdev | 733 | 1050 | 733 | 877 | 733 | 270 |
| p(t-test) | | 0.0093 | | 0.047 | | 0.91 |
| Min | 86.8 | 231 | 86.8 | 231 | 86.8 | 231 |
| Max | 3370 | 3200 | 3370 | 3200 | 3370 | 932 |
| n (Samp) | 90 | 11 | 90 | 11 | 90 | 6 |
| n (Patient) | 90 | 11 | 90 | 11 | 90 | 6 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 338 | 655 | 338 | 655 | 338 | 655 |
| Average | 707 | 576 | 707 | 576 | 707 | 576 |
| Stdev | 844 | 270 | 844 | 270 | 844 | 270 |
| p(t-test) | | 0.70 | | 0.70 | | 0.70 |
| Min | 86.8 | 231 | 86.8 | 231 | 86.8 | 231 |
| Max | 3370 | 932 | 3370 | 932 | 3370 | 932 |
| n (Samp) | 178 | 6 | 178 | 6 | 178 | 6 |
| n (Patient) | 178 | 6 | 178 | 6 | 178 | 6 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 355 | 1390 | 355 | 1280 | nd | nd |
| Average | 632 | 1680 | 632 | 1410 | nd | nd |
| Stdev | 702 | 1110 | 702 | 948 | nd | nd |
| p(t-test) | | 4.0E-4 | | 0.0068 | nd | nd |
| Min | 86.8 | 618 | 86.8 | 618 | nd | nd |
| Max | 3370 | 3200 | 3370 | 3200 | nd | nd |
| n (Samp) | 94 | 7 | 94 | 7 | nd | nd |
| n (Patient) | 94 | 7 | 94 | 7 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.76 | 0.62 | 0.85 | 0.75 | 0.62 | 0.83 | 0.64 | 0.62 | nd |
| SE | 0.087 | 0.12 | 0.093 | 0.088 | 0.12 | 0.097 | 0.13 | 0.12 | nd |
| p | 0.0029 | 0.35 | 1.8E-4 | 0.0046 | 0.35 | 7.3E-4 | 0.25 | 0.35 | nd |
| nCohort 1 | 90 | 178 | 94 | 90 | 178 | 94 | 90 | 178 | nd |
| nCohort 2 | 11 | 6 | 7 | 11 | 6 | 7 | 6 | 6 | nd |
| Cutoff 1 | 626 | 280 | 689 | 626 | 280 | 689 | 278 | 280 | nd |
| Sens 1 | 73% | 83% | 71% | 73% | 83% | 71% | 83% | 83% | nd |
| Spec 1 | 74% | 43% | 74% | 74% | 43% | 74% | 42% | 43% | nd |
| Cutoff 2 | 591 | 280 | 626 | 591 | 280 | 626 | 278 | 280 | nd |
| Sens 2 | 82% | 83% | 86% | 82% | 83% | 86% | 83% | 83% | nd |
| Spec 2 | 73% | 43% | 71% | 73% | 43% | 71% | 42% | 43% | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 3 | 278 | 228 | 591 | 278 | 228 | 591 | 228 | 228 | nd |
| Sens 3 | 91% | 100% | 100% | 91% | 100% | 100% | 100% | 100% | nd |
| Spec 3 | 42% | 30% | 70% | 42% | 30% | 70% | 34% | 30% | nd |
| Cutoff 4 | 475 | 578 | 591 | 475 | 578 | 591 | 475 | 578 | nd |
| Sens 4 | 82% | 67% | 100% | 82% | 67% | 100% | 67% | 67% | nd |
| Spec 4 | 70% | 70% | 70% | 70% | 70% | 70% | 70% | 70% | nd |
| Cutoff 5 | 754 | 946 | 933 | 754 | 946 | 933 | 754 | 946 | nd |
| Sens 5 | 45% | 0% | 57% | 45% | 0% | 57% | 17% | 0% | nd |
| Spec 5 | 80% | 80% | 81% | 80% | 80% | 81% | 80% | 80% | nd |
| Cutoff 6 | 1660 | 2040 | 1810 | 1660 | 2040 | 1810 | 1660 | 2040 | nd |
| Sens 6 | 27% | 0% | 43% | 18% | 0% | 29% | 0% | 0% | nd |
| Spec 6 | 90% | 90% | 91% | 90% | 90% | 91% | 90% | 90% | nd |
| OR Quart 2 | >2.2 | >2.1 | >0 | >2.2 | >2.1 | >0 | >2.2 | >2.1 | nd |
| p Value | <0.54 | <0.55 | <na | <0.54 | <0.55 | <na | <0.54 | <0.55 | nd |
| 95% CI of | >0.18 | >0.18 | >na | >0.18 | >0.18 | >na | >0.18 | >0.18 | nd |
| OR Quart2 | na | na | na | na | na | na | na | na | nd |
| OR Quart 3 | >4.8 | >3.2 | >3.4 | >4.8 | >3.2 | >3.4 | >1.0 | >3.2 | nd |
| p Value | <0.18 | <0.32 | <0.30 | <0.18 | <0.32 | <0.30 | <0.98 | <0.32 | nd |
| 95% CI of | >0.49 | >0.32 | >0.33 | >0.49 | >0.32 | >0.33 | >0.062 | >0.32 | nd |
| OR Quart3 | na | na | na | na | na | na | na | na | nd |
| OR Quart 4 | >6.0 | >1.0 | >4.5 | >6.0 | >1.0 | >4.5 | >3.4 | >1.0 | nd |
| p Value | <0.12 | <0.99 | <0.19 | <0.12 | <0.99 | <0.19 | <0.30 | <0.99 | nd |
| 95% CI of | >0.64 | >0.062 | >0.47 | >0.64 | >0.062 | >0.47 | >0.33 | >0.062 | nd |
| OR Quart4 | na | na | na | na | na | na | na | na | nd |

Table 9: Comparison of marker levels in urine samples collected from
Cohort 1 (patients that did not progress beyond RIFLE stage 0, R, or I) and in
urine samples collected from Cohort 2 (subjects who progress to RIFLE stage F)
at 0, 24 hours, and 48 hours prior to the subject reaching RIFLE stage I.

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1300 | 2590 | 1300 | 3200 | 1300 | 2010 |
| Average | 1670 | 2900 | 1670 | 3320 | 1670 | 2430 |
| Stdev | 1300 | 1820 | 1300 | 1750 | 1300 | 1860 |
| p(t-test) | | 1.9E-7 | | 4.1E-11 | | 0.020 |
| Min | 41.6 | 390 | 41.6 | 687 | 41.6 | 81.2 |
| Max | 6400 | 6400 | 6400 | 6400 | 6400 | 6190 |
| n (Samp) | 1703 | 31 | 1703 | 28 | 1703 | 16 |
| n (Patient) | 580 | 31 | 580 | 28 | 580 | 16 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1360 | 2480 | 1360 | 2500 | 1360 | 1880 |
| Average | 1750 | 2480 | 1750 | 2860 | 1750 | 2240 |
| Stdev | 1390 | 1900 | 1390 | 1150 | 1390 | 1120 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| p(t-test) | | 0.083 | | 0.012 | | 0.30 |
| Min | 41.6 | 565 | 41.6 | 1430 | 41.6 | 1040 |
| Max | 6400 | 6400 | 6400 | 5000 | 6400 | 4360 |
| n (Samp) | 1782 | 11 | 1782 | 10 | 1782 | 9 |
| n (Patient) | 600 | 11 | 600 | 10 | 600 | 9 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1380 | 3210 | 1380 | 3220 | 1380 | 3480 |
| Average | 1720 | 3380 | 1720 | 3530 | 1720 | 3040 |
| Stdev | 1300 | 1950 | 1300 | 1930 | 1300 | 2330 |
| p(t-test) | | 2.4E-8 | | 1.1E-11 | | 0.0045 |
| Min | 41.6 | 390 | 41.6 | 687 | 41.6 | 379 |
| Max | 6400 | 6400 | 6400 | 6400 | 6400 | 6190 |
| n (Samp) | 1587 | 20 | 1587 | 25 | 1587 | 8 |
| n (Patient) | 499 | 20 | 499 | 25 | 499 | 8 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.71 | 0.62 | 0.75 | 0.78 | 0.78 | 0.78 | 0.61 | 0.67 | 0.63 |
| SE | 0.053 | 0.091 | 0.063 | 0.052 | 0.087 | 0.055 | 0.075 | 0.100 | 0.11 |
| p | 7.5E-5 | 0.20 | 7.1E-5 | 4.9E-8 | 0.0015 | 5.5E-7 | 0.14 | 0.097 | 0.22 |
| nCohort 1 | 1703 | 1782 | 1587 | 1703 | 1782 | 1587 | 1703 | 1782 | 1587 |
| nCohort 2 | 31 | 11 | 20 | 28 | 10 | 25 | 16 | 9 | 8 |
| Cutoff 1 | 1660 | 1070 | 2470 | 2450 | 2270 | 2450 | 1040 | 1420 | 480 |
| Sens 1 | 71% | 73% | 70% | 71% | 70% | 72% | 75% | 78% | 75% |
| Spec 1 | 62% | 40% | 78% | 79% | 74% | 78% | 39% | 52% | 14% |
| Cutoff 2 | 1030 | 934 | 1660 | 1440 | 2010 | 1540 | 480 | 1310 | 471 |
| Sens 2 | 81% | 82% | 80% | 82% | 80% | 80% | 81% | 89% | 88% |
| Spec 2 | 39% | 34% | 60% | 55% | 68% | 56% | 15% | 49% | 13% |
| Cutoff 3 | 874 | 577 | 874 | 819 | 1960 | 808 | 378 | 1040 | 378 |
| Sens 3 | 90% | 91% | 90% | 93% | 90% | 92% | 94% | 100% | 100% |
| Spec 3 | 32% | 19% | 30% | 30% | 67% | 28% | 10% | 38% | 9% |
| Cutoff 4 | 1990 | 2070 | 2050 | 1990 | 2070 | 2050 | 1990 | 2070 | 2050 |
| Sens 4 | 65% | 55% | 70% | 75% | 70% | 76% | 50% | 44% | 62% |
| Spec 4 | 70% | 70% | 70% | 70% | 70% | 70% | 70% | 70% | 70% |
| Cutoff 5 | 2560 | 2710 | 2650 | 2560 | 2710 | 2650 | 2560 | 2710 | 2650 |
| Sens 5 | 52% | 36% | 55% | 61% | 40% | 64% | 38% | 22% | 62% |
| Spec 5 | 80% | 80% | 80% | 80% | 80% | 80% | 80% | 80% | 80% |
| Cutoff 6 | 3620 | 3850 | 3690 | 3620 | 3850 | 3690 | 3620 | 3850 | 3690 |
| Sens 6 | 29% | 18% | 45% | 39% | 20% | 48% | 25% | 11% | 38% |
| Spec 6 | 90% | 90% | 90% | 90% | 90% | 90% | 90% | 90% | 90% |
| OR Quart 2 | 1.7 | 1.0 | 3.0 | 2.0 | >0 | 4.0 | 0.25 | >2.0 | 0 |
| p Value | 0.48 | 1.0 | 0.34 | 0.57 | <na | 0.21 | 0.21 | <0.57 | na |
| 95% CI of | 0.40 | 0.14 | 0.31 | 0.18 | >na | 0.45 | 0.028 | >0.18 | na |
| OR Quart2 | 7.0 | 7.1 | 29 | 22 | na | 36 | 2.2 | na | na |
| OR Quart 3 | 1.3 | 0.50 | 2.0 | 5.0 | >4.0 | 2.0 | 1.00 | >4.0 | 0 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| p Value | 0.71 | 0.57 | 0.57 | 0.14 | <0.21 | 0.57 | 1.00 | <0.21 | na |
| 95% CI of | 0.30 | 0.045 | 0.18 | 0.59 | >0.45 | 0.18 | 0.25 | >0.45 | na |
| OR Quart3 | 6.0 | 5.5 | 22 | 43 | na | 22 | 4.0 | na | na |
| OR Quart 4 | 6.6 | 3.0 | 14 | 21 | >6.1 | 19 | 1.8 | >3.0 | 1.7 |
| p Value | 0.0026 | 0.18 | 0.010 | 0.0031 | <0.095 | 0.0044 | 0.37 | <0.34 | 0.48 |
| 95% CI of | 1.9 | 0.61 | 1.9 | 2.8 | >0.73 | 2.5 | 0.51 | >0.31 | 0.40 |
| OR Quart4 | 22 | 15 | 110 | 160 | na | 140 | 6.1 | na | 7.0 |

Table 10: Comparison of marker levels in EDTA samples collected from Cohort 1 (patients that did not progress beyond RIFLE stage 0, R, or I) and in EDTA samples collected from Cohort 2 (subjects who progress to RIFLE stage F) at 0, 24 hours, and 48 hours prior to the subject reaching RIFLE stage I.

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 326 | 618 | nd | nd |
| Average | nd | nd | 606 | 1130 | nd | nd |
| Stdev | nd | nd | 706 | 1140 | nd | nd |
| p(t-test) | nd | nd | | 0.054 | nd | nd |
| Min | nd | nd | 48.0 | 190 | nd | nd |
| Max | nd | nd | 3370 | 3200 | nd | nd |
| n (Samp) | nd | nd | 489 | 7 | nd | nd |
| n (Patient) | nd | nd | 222 | 7 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 326 | 1000 | nd | nd |
| Average | nd | nd | 604 | 1340 | nd | nd |
| Stdev | nd | nd | 698 | 1110 | nd | nd |
| p(t-test) | nd | nd | | 0.011 | nd | nd |
| Min | nd | nd | 48.0 | 279 | nd | nd |
| Max | nd | nd | 3370 | 3200 | nd | nd |
| n (Samp) | nd | nd | 485 | 6 | nd | nd |
| n (Patient) | nd | nd | 208 | 6 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | nd | nd | nd | 0.64 | nd | 0.78 | nd | nd | nd |
| SE | nd | nd | nd | 0.11 | nd | 0.11 | nd | nd | nd |
| p | nd | nd | nd | 0.21 | nd | 0.012 | nd | nd | nd |
| nCohort 1 | nd | nd | nd | 489 | nd | 485 | nd | nd | nd |
| nCohort 2 | nd | nd | nd | 7 | nd | 6 | nd | nd | nd |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| Cutoff 1 | nd | nd | nd | 278 | nd | 560 | nd | nd | nd |
| Sens 1 | nd | nd | nd | 71% | nd | 83% | nd | nd | nd |
| Spec 1 | nd | nd | nd | 42% | nd | 73% | nd | nd | nd |
| Cutoff 2 | nd | nd | nd | 228 | nd | 560 | nd | nd | nd |
| Sens 2 | nd | nd | nd | 86% | nd | 83% | nd | nd | nd |
| Spec 2 | nd | nd | nd | 31% | nd | 73% | nd | nd | nd |
| Cutoff 3 | nd | nd | nd | 190 | nd | 278 | nd | nd | nd |
| Sens 3 | nd | nd | nd | 100% | nd | 100% | nd | nd | nd |
| Spec 3 | nd | nd | nd | 22% | nd | 42% | nd | nd | nd |
| Cutoff 4 | nd | nd | nd | 515 | nd | 518 | nd | nd | nd |
| Sens 4 | nd | nd | nd | 57% | nd | 83% | nd | nd | nd |
| Spec 4 | nd | nd | nd | 70% | nd | 70% | nd | nd | nd |
| Cutoff 5 | nd | nd | nd | 845 | nd | 833 | nd | nd | nd |
| Sens 5 | nd | nd | nd | 43% | nd | 50% | nd | nd | nd |
| Spec 5 | nd | nd | nd | 80% | nd | 80% | nd | nd | nd |
| Cutoff 6 | nd | nd | nd | 1670 | nd | 1660 | nd | nd | nd |
| Sens 6 | nd | nd | nd | 29% | nd | 33% | nd | nd | nd |
| Spec 6 | nd | nd | nd | 90% | nd | 90% | nd | nd | nd |
| OR Quart 2 | nd | nd | nd | 2.0 | nd | >1.0 | nd | nd | nd |
| p Value | nd | nd | nd | 0.57 | nd | <1.0 | nd | nd | nd |
| 95% CI of | nd | nd | nd | 0.18 | nd | >0.062 | nd | nd | nd |
| OR Quart2 | nd | nd | nd | 23 | nd | na | nd | nd | nd |
| OR Quart 3 | nd | nd | nd | 1.0 | nd | >2.0 | nd | nd | nd |
| p Value | nd | nd | nd | 1.0 | nd | <0.57 | nd | nd | nd |
| 95% CI of | nd | nd | nd | 0.062 | nd | >0.18 | nd | nd | nd |
| OR Quart3 | nd | nd | nd | 16 | nd | na | nd | nd | nd |
| OR Quart 4 | nd | nd | nd | 3.0 | nd | >3.0 | nd | nd | nd |
| p Value | nd | nd | nd | 0.34 | nd | <0.34 | nd | nd | nd |
| 95% CI of | nd | nd | nd | 0.31 | nd | >0.31 | nd | nd | nd |
| OR Quart4 | nd | nd | nd | 30 | nd | na | nd | nd | nd |

Table 11: Comparison of marker levels in enroll urine samples collected from Cohort 1 (patients that did not progress beyond RIFLE stage 0 or R within 48hrs) and in enroll urine samples collected from Cohort 2 (subjects reaching RIFLE stage I or F within 48hrs). Enroll samples from patients already at RIFLE stage I or F were included in Cohort 2.

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| Median | 1170 | 2300 | 1260 | 2800 | 1220 | 2220 |
| Average | 1480 | 2660 | 1680 | 2830 | 1560 | 2680 |
| Stdev | 1160 | 1770 | 1360 | 1780 | 1180 | 1810 |
| p(t-test) | | 2.1E-18 | | 2.0E-5 | | 3.6E-14 |
| Min | 41.6 | 81.2 | 41.6 | 197 | 41.6 | 81.2 |
| Max | 6300 | 6400 | 6400 | 6390 | 5430 | 6400 |
| n (Samp) | 484 | 129 | 576 | 28 | 406 | 110 |

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| n (Patient) | 484 | 129 | 576 | 28 | 406 | 110 |

| | At Enrollment | | |
|---|---|---|---|
| | sCr or UO | sCr only | UO only |
| AUC | 0.70 | 0.69 | 0.69 |
| SE | 0.028 | 0.057 | 0.030 |
| p | 3.5E-13 | 7.6E-4 | 6.8E-10 |
| nCohort 1 | 484 | 576 | 406 |
| nCohort 2 | 129 | 28 | 110 |
| Cutoff 1 | 1380 | 1450 | 1380 |
| Sens 1 | 71% | 71% | 70% |
| Spec 1 | 58% | 57% | 56% |
| Cutoff 2 | 886 | 808 | 949 |
| Sens 2 | 81% | 82% | 80% |
| Spec 2 | 38% | 32% | 38% |
| Cutoff 3 | 616 | 551 | 674 |
| Sens 3 | 91% | 93% | 90% |
| Spec 3 | 24% | 19% | 25% |
| Cutoff 4 | 1760 | 1990 | 1880 |
| Sens 4 | 59% | 68% | 57% |
| Spec 4 | 70% | 70% | 70% |
| Cutoff 5 | 2280 | 2660 | 2440 |
| Sens 5 | 50% | 61% | 46% |
| Spec 5 | 80% | 80% | 80% |
| Cutoff 6 | 3190 | 3790 | 3310 |
| Sens 6 | 36% | 29% | 34% |
| Spec 6 | 90% | 90% | 90% |
| OR Quart 2 | 1.1 | 0.39 | 1.1 |
| p Value | 0.86 | 0.27 | 0.85 |
| 95% CI of | 0.53 | 0.075 | 0.52 |
| OR Quart2 | 2.2 | 2.1 | 2.2 |
| OR Quart 3 | 2.0 | 0.79 | 1.9 |
| p Value | 0.030 | 0.74 | 0.069 |
| 95% CI of | 1.1 | 0.21 | 0.95 |
| OR Quart3 | 3.9 | 3.0 | 3.7 |
| OR Quart 4 | 5.5 | 3.7 | 4.5 |
| p Value | 2.0E-8 | 0.012 | 3.4E-6 |
| 95% CI of | 3.0 | 1.3 | 2.4 |
| OR Quart4 | 10 | 10 | 8.4 |

Table 12: Comparison of marker levels in enroll EDTA samples collected from Cohort 1 (patients that did not progress beyond RIFLE stage 0 or R within 48hrs) and in enroll EDTA samples collected from Cohort 2 (subjects reaching RIFLE stage I or F within 48hrs). Enroll samples from patients already at stage I or F were included in Cohort 2.

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 309 | 266 | nd | nd | 354 | 247 |
| Average | 651 | 674 | nd | nd | 647 | 679 |
| Stdev | 791 | 841 | nd | nd | 774 | 856 |
| p(t-test) |  | 0.89 | nd | nd |  | 0.85 |
| Min | 76.0 | 48.0 | nd | nd | 76.0 | 48.0 |
| Max | 3350 | 3200 | nd | nd | 3350 | 3200 |
| n (Samp) | 140 | 29 | nd | nd | 133 | 28 |
| n (Patient) | 140 | 29 | nd | nd | 133 | 28 |

|  | At Enrollment | | |
|---|---|---|---|
|  | sCr or UO | sCr only | UO only |
| AUC | 0.48 | nd | 0.47 |
| SE | 0.059 | nd | 0.061 |
| p | 0.79 | nd | 0.61 |
| nCohort 1 | 140 | nd | 133 |
| nCohort 2 | 29 | nd | 28 |
| Cutoff 1 | 184 | nd | 184 |
| Sens 1 | 72% | nd | 71% |
| Spec 1 | 23% | nd | 21% |
| Cutoff 2 | 140 | nd | 140 |
| Sens 2 | 83% | nd | 82% |
| Spec 2 | 14% | nd | 11% |
| Cutoff 3 | 93.7 | nd | 93.7 |
| Sens 3 | 93% | nd | 93% |
| Spec 3 | 3% | nd | 3% |
| Cutoff 4 | 517 | nd | 538 |
| Sens 4 | 41% | nd | 36% |
| Spec 4 | 70% | nd | 71% |
| Cutoff 5 | 882 | nd | 882 |
| Sens 5 | 21% | nd | 21% |
| Spec 5 | 80% | nd | 80% |
| Cutoff 6 | 1860 | nd | 1860 |
| Sens 6 | 10% | nd | 11% |
| Spec 6 | 90% | nd | 90% |
| OR Quart 2 | 1.5 | nd | 1.2 |
| p Value | 0.53 | nd | 0.73 |
| 95% CI of | 0.46 | nd | 0.38 |
| OR Quart2 | 4.6 | nd | 4.1 |
| OR Quart 3 | 1.0 | nd | 1.0 |
| p Value | 0.96 | nd | 0.96 |
| 95% CI of | 0.30 | nd | 0.30 |
| OR Quart3 | 3.5 | nd | 3.5 |
| OR Quart 4 | 1.7 | nd | 1.7 |
| p Value | 0.37 | nd | 0.37 |
| 95% CI of | 0.54 | nd | 0.54 |
| OR Quart4 | 5.2 | nd | 5.3 |

Publications:

1. Uchino S, Kellum JA, Bellomo R, Doig GS, Morimatsu H, Morgera S, Schetz M, Tan I, Bouman C, Macedo E, Gibney N, Tolwani A, Ronco C: Acute renal failure in critically ill patients: a multinational, multicenter study, Jama 2005,294:813-818

2. Manns B, Doig CJ, Lee H, Dean S, Tonelli M, Johnson D, Donaldson C: Cost of acute renal failure requiring dialysis in the intensive care unit: clinical and resource implications of renal recovery, Crit Care Med 2003, 31:449-455

3. Hansell P, Goransson V, Odlind C, Gerdin B, Hallgren R: Hyaluronan content in the kidney in different states of body hydration, Kidney Int 2000, 58:2061-2068

4. Sibalic V, Fan X, Loffing J, Wuthrich RP: Upregulated renal tubular CD44, hyaluronan, and osteopontin in kdkd mice with interstitial nephritis, Nephrol Dial Transplant 1997, 12:1344-1353

5. Lewington AJ, Padanilam BJ, Martin DR, Hammerman MR: Expression of CD44 in kidney after acute ischemic injury in rats, Am J Physiol Regul Integr Comp Physiol 2000, 278:R247-254

6. Sano N, Kitazawa K, Sugisaki T: Localization and roles of CD44, hyaluronic acid and osteopontin in IgA nephropathy, Nephron 2001, 89:416-421

7. Melin J, Hellberg O, Funa K, Hallgren R, Larsson E, Fellstrom BC: Ischemia-induced renal expression of hyaluronan and CD44 in diabetic rats, Nephron Exp Nephrol 2006, 103:e86-94

8. Yang J, Liu Y: Dissection of key events in tubular epithelial to myofibroblast transition and its implications in renal interstitial fibrosis, Am J Pathol 2001, 159:1465-1475

9. Okajima K: Regulation of inflammatory responses by natural anticoagulants, Immunol Rev 2001, 184:258-274

10. Wang X, Huang G, Mei S, Qian J, Ji J, Zhang J: Over-expression of C/EBP-alpha induces apoptosis in cultured rat hepatic stellate cells depending on p53 and peroxisome proliferator-activated receptor-gamma, Biochem Biophys Res Commun 2009, 380:286-291

11. Takeda K, Kojima Y, Ikejima K, Harada K, Yamashina S, Okumura K, Aoyama T, Frese S, Ikeda H, Haynes NM, Cretney E, Yagita H, Sueyoshi N, Sato N, Nakanuma Y, Smyth MJ, Okumura K: Death receptor 5 mediated-apoptosis contributes to cholestatic liver disease, Proc Natl Acad Sci U S A 2008, 105:10895-10900

12. Wolf G: Renal injury due to renin-angiotensin-aldosterone system activation of the transforming growth factor-beta pathway, Kidney Int 2006, 70:1914-1919

13. Basile DP: The endothelial cell in ischemic acute kidney injury: implications for acute and chronic function, Kidney Int 2007, 72:151-156

14. Hvidberg V, Jacobsen C, Strong RK, Cowland JB, Moestrup SK, Borregaard N: The endocytic receptor megalin binds the iron transporting neutrophil-gelatinase-associated lipocalin with high affinity and mediates its cellular uptake, FEBS Lett 2005, 579:773-777

15. Mori K, Nakao K: Neutrophil gelatinase-associated lipocalin as the real-time indicator of active kidney damage, Kidney Int 2007, 71:967-970

16. Mori K, Lee HT, Rapoport D, Drexler IR, Foster K, Yang J, Schmidt-Ott KM, Chen X, Li JY, Weiss S, Mishra J, Cheema FH, Markowitz G, Suganami T, Sawai K, Mukoyama M, Kunis C, D'Agati V, Devarajan P, Barasch J: Endocytic delivery of lipocalin-siderophore-iron complex rescues the kidney from ischemia-reperfusion injury, J Clin Invest 2005, 115:610-621

17. Nickolas TL, O'Rourke MJ, Yang J, Sise ME, Canetta PA, Barasch N, Buchen C, Khan F, Mori K, Giglio J, Devarajan P, Barasch J: Sensitivity and specificity of a single emergency department measurement of urinary neutrophil gelatinase-associated lipocalin for diagnosing acute kidney injury, Ann Intern Med 2008, 148:810-819

18. Palevsky PM, Zhang JH, O'Connor TZ, Chertow GM, Crowley ST, Choudhury D, Finkel K, Kellum JA, Paganini E, Schein RM, Smith MW, Swanson KM, Thompson BT, Vijayan A, Watnick S, Star RA, Peduzzi P: Intensity of

# EP 2 619 231 B1

renal support in critically ill patients with acute kidney injury, N Engl J Med 2008, 359:7-20

19. Bone RC, Balk RA, Cerra FB, Dellinger RP, Fein AM, Knaus WA, Schein RM, Sibbald WJ: Definitions for sepsis and organ failure and guidelines for the use of innovative therapies in sepsis. The ACCP/SCCM Consensus Conference Committee. American College of Chest Physicians/Society of Critical Care Medicine. 1992, Chest 2009, 136:e28

20. Bellomo R, Ronco C, Kellum JA, Mehta RL, Palevsky P: Acute renal failure - definition, outcome measures, animal models, fluid therapy and information technology needs: the Second International Consensus Conference of the Acute Dialysis Quality Initiative (ADQI) Group, Crit Care 2004, 8:R204-212

21. Ronco P, Lelongt B, Piedagnel R, Chatziantoniou C: Matrix metalloproteinases in kidney disease progression and repair: a case of flipping the coin, Semin Nephrol 2007, 27:352-362

## Claims

1.  A method for evaluating renal status in a subject not receiving renal replacement therapy, comprising:

    performing one or more assays configured to detect one or more biomarkers, the biomarkers comprising hyaluronic acid (HA), on a body fluid sample obtained from the subject to provide one or more assay results; and correlating the assay result(s) to one or more of risk stratification to identify subjects at risk for a future injury to renal function, for future progression to reduced renal function, for future progression to ARF and prognosis of the renal status of the subject, wherein the subject is not in acute renal failure and wherein the subject (i) has not experienced a 1.5-fold or greater increase in serum creatinine over a baseline value determined prior to the time at which the body fluid sample is obtained, and/or (iii) has not experienced an increase of 0.3 mg/dL or greater in serum creatinine over a baseline value determined prior to the time at which the body fluid sample is obtained.

2.  A method according to claim 1, wherein the subject is in RIFLE stage 0.

3.  A method according to claim 2, wherein the subject is in RIFLE stage 0, and said correlating step comprises assigning a likelihood that the subject will reach RIFLE stage R, I or F within 72 hours, within 48 hours or within 24 hours, wherein said assay result(s) optionally comprise a measured urine concentration of HA and said correlation step comprises comparing said measure concentration to a threshold concentration, and assigning an increased likelihood of progressing to RIFLE stage R, I or F to the subject, when the measured concentration is above the threshold, or assigning a decreased likelihood of progressing to RIFLE stage R, I or F to the subject when the measured concentration is below the threshold.

4.  A method according to claim 2, wherein the subject is in RIFLE stage 0, and said correlating step comprises assigning a likelihood that the subject will reach RIFLE stage I or F within 72 hours, within 48 hours or within 24 hours, wherein

    (i) said assay result(s) optionally comprise a measured urine concentration of HA and said correlation step comprises comparing said measure concentration to a threshold concentration, and assigning an increased likelihood of progressing to RIFLE stage I or F to the subject, when the measured concentration is above the threshold, or assigning a decreased likelihood of progressing to RIFLE stage I or F to the subject when the measured concentration is below the threshold or
    (ii) the subject is optionally in RIFLE stage 0, and said correlating step comprises assigning a likelihood that the subject will reach RIFLE stage F within 72 hours, within 48 hours or within 24 hours,

    wherein said assay result(s) optionally comprise a measured urine concentration of HA and said correlation step comprises comparing said measure concentration to a threshold concentration, and assigning an increased likelihood of progressing to RIFLE stage F to the subject, when the measured concentration is above the threshold, or assigning a decreased likelihood of progressing to RIFLE stage F to the subject when the measured concentration is below the threshold.

5.  A method according to claim 1, wherein said assay result(s) comprise

(a) a measured urine concentration of HA and said correlation step comprises comparing said measure concentration to a threshold concentration, and

assigning an increased likelihood of progressing to a worsening RIFLE stage to the subject, relative to the subject's current RIFLE stage, when the measured concentration is above the threshold, or assigning a decreased likelihood of progressing to a worsening RIFLE stage to the subject, relative to the subject's current RIFLE stage, when the measured concentration is below the threshold,

(b) a measured urine concentration of HA and said correlation step comprises comparing said measure concentration to a threshold concentration, and

assigning an increased likelihood of progressing to a need for renal replacement therapy to the subject when the measured concentration is above the threshold, or assigning a decreased likelihood of progressing to a need for renal replacement therapy when the measured concentration is below the threshold,

(c) a measured urine concentration of HA and said correlation step comprises comparing said measure concentration to a threshold concentration, and

assigning an increased likelihood of progressing to a worsening RIFLE stage to the subject, relative to the subject's current RIFLE stage, when the measured concentration is above the threshold, or assigning a decreased likelihood of progressing to a worsening RIFLE stage to the subject, relative to the subject's current RIFLE stage, when the measured concentration is below the threshold, or

(d) a measured urine concentration of HA and said correlation step comprises comparing said measure concentration to a threshold concentration, and

assigning an increased likelihood of progressing to acute renal failure when the measured concentration is above the threshold, or assigning a decreased likelihood of progressing to acute renal failure to the subject when the measured concentration is below the threshold.

6. A method according to claim 1, wherein the subject is selected for evaluation of renal status based on

(a) the pre-existence in the subject of one or more known risk factors for prerenal, intrinsic renal, or postrenal ARF or

(b) an existing diagnosis of one or more of congestive heart failure, preeclampsia, eclampsia, diabetes mellitus, hypertension, coronary artery disease, proteinuria, renal insufficiency, glomerular filtration below the normal range, cirrhosis, serum creatinine above the normal range, sepsis, injury to renal function, reduced renal function, or ARF, or based on undergoing or having undergone major vascular surgery, coronary artery bypass, or other cardiac surgery, or based on exposure to NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin.

7. The use of HA for the risk stratification to identify subjects at risk for a future injury to renal function, for future progression to reduced renal function, for future progression to ARF and prognosis of renal status of a subject not receiving renal replacement therapy or a subject not in acute renal failure, or for assigning an increased likelihood of progressing to a worsening RIFLE stage to a subject, relative to the subject's current RIFLE stage, wherein the subject (i) has not experienced a 1.5-fold or greater increase in serum creatinine over a baseline value determined prior to the time at which the body fluid sample is obtained, and/or (iii) has not experienced an increase of 0.3 mg/dL or greater in serum creatinine over a baseline value determined prior to the time at which the body fluid sample is obtained, the use comprising an urine measurement of HA.

**Patentansprüche**

1. Ein Verfahren zum Evaluieren des Nierenstatus bei einem Subjekt, das keine Nierenersatztherapie erhält, umfassend:

Durchführen einer oder mehrerer Untersuchungen, die dafür konfiguriert sind einen oder mehrere Biomarker, wobei die Biomarker Hyaluronsäure (HA) umfassen, in einer von dem Subjekt erhaltenen Körperflüssigkeitsprobe nachzuweisen, um ein oder mehrere Untersuchungsergebnisse bereitzustellen; und

Korrelieren des/der Untersuchungsergebnisse(s) mit einem oder mehreren aus Risikostratifizierung zum Identifizieren von Subjekten, die von zukünftiger Verletzung der Nierenfunktion, von zukünftigem Fortschreiten zu einer verringerten Nierenfunktion, von zukünftigem Fortschreiten zu ARF gefährdet sind und Prognose des Nierenstatus des Subjektes, wobei das Subjekt kein akutes Nierenversagen hat und wobei das Subjekt (i) keinen 1,5-fachen oder höheren Anstieg von Serumkreatinin über einem Ausgangswert, der vor dem Zeitpunkt, an dem die Körperflüssigkeitsprobe erhalten wurde, bestimmt wurde, erfahren hat und/oder (iii) keinen Anstieg von Serumkreatinin von 0,3 mg/dl oder mehr über einen Ausgangswert, der vor dem Zeitpunkt, an dem die Körperflüssigkeitsprobe erhalten wurde, bestimmt wurde, erfahren hat.

2. Ein Verfahren nach Anspruch 1, wobei das Subjekt in RIFLE-Stadium 0 ist.

3. Ein Verfahren nach Anspruch 2, wobei das Subjekt in RIFLE-Stadium 0 ist und der Korrelationsschritt das Zuordnen einer Wahrscheinlichkeit, dass das Subjekt RIFLE-Stadium R, I oder F innerhalb von 72 Stunden, innerhalb von 48 Stunden oder innerhalb von 24 Stunden erreichen wird, umfasst,
wobei das/die Untersuchungsergebnis(se) optional eine gemessene Urinkonzentration von HA umfassen und der Korrelationsschritt das Vergleichen der gemessenen Konzentration mit einer Schwellenkonzentration umfasst und Zuordnen einer erhöhten Wahrscheinlichkeit des Fortschreitens zu RIFLE-Stadium R, I oder F zu dem Subjekt, wenn die gemessene Konzentration über dem Schwellenwert liegt oder Zuordnen einer verringerten Wahrscheinlichkeit des Fortschreitens zu RIFLE-Stadium R, I oder F zu dem Subjekt, wenn die gemessene Konzentration unter dem Schwellenwert liegt.

4. Ein Verfahren nach Anspruch 2, wobei das Subjekt in RIFLE-Stadium 0 ist und der Korrelationsschritt das Zuordnen einer Wahrscheinlichkeit, dass das Subjekt RIFLE-Stadium I oder F innerhalb von 72 Stunden, innerhalb von 48 Stunden oder innerhalb von 24 Stunden erreichen wird, umfasst, wobei

(i) das/die Untersuchungsergebnis(se) optional eine gemessene Urinkonzentration von HA umfassen und der Korrelationsschritt das Vergleichen der gemessenen Konzentration mit einer Schwellenkonzentration umfasst und
Zuordnen einer erhöhten Wahrscheinlichkeit des Fortschreitens zu RIFLE-Stadium I oder F zu dem Subjekt, wenn die gemessene Konzentration über dem Schwellenwert liegt oder Zuordnen einer verringerten Wahrscheinlichkeit des Fortschreitens zu RIFLE-Stadium I oder F zu dem Subjekt, wenn die gemessene Konzentration unter dem Schwellenwert liegt oder
(ii) das Subjekt optional in RIFLE-Stadium 0 ist und der Korrelationsschritt das Zuordnen einer Wahrscheinlichkeit, dass das Subjekt RIFLE-Stadium F innerhalb von 72 Stunden, innerhalb von 48 Stunden oder innerhalb von 24 Stunden erreichen wird, umfasst,

wobei das/die Untersuchungsergebnis(se) optional eine gemessene Urinkonzentration von HA umfassen und der Korrelationsschritt das Vergleichen der gemessenen Konzentration mit einer Schwellenkonzentration umfasst und Zuordnen einer erhöhten Wahrscheinlichkeit des Fortschreitens zu RIFLE-Stadium F zu dem Subjekt, wenn die gemessene Konzentration über dem Schwellenwert liegt oder Zuordnen einer verringerten Wahrscheinlichkeit des Fortschreitens zu RIFLE-Stadium F zu dem Subjekt, wenn die gemessene Konzentration unter dem Schwellenwert liegt.

5. Ein Verfahren nach Anspruch 1, wobei das/die Untersuchungsergebnis(se) umfassen

(a) eine gemessene Urinkonzentration von HA und wobei der Korrelationsschritt das Vergleichen der gemessenen Konzentration mit einer Schwellenkonzentration umfasst und Zuordnen einer erhöhten Wahrscheinlichkeit des Fortschreitens zu einem schlechteren RIFLE-Stadium zu dem Subjekt, in Bezug auf das derzeitige RIFLE-Stadium des Subjektes, wenn die gemessene Konzentration über dem Schwellenwert liegt oder Zuordnen einer verringerten Wahrscheinlichkeit des Fortschreitens zu einem schlechteren RIFLE-Stadium zu dem Subjekt, in Bezug auf das derzeitige RIFLE-Stadium des Subjektes, wenn die gemessene Konzentration unter dem Schwellenwert liegt,
(b) eine gemessene Urinkonzentration von HA und wobei der Korrelationsschritt das Vergleichen der gemessenen Konzentration mit einer Schwellenkonzentration umfasst und Zuordnen einer erhöhten Wahrscheinlichkeit des Fortschreitens zu einer Notwendigkeit einer Nierenersatztherapie zu dem Subjekt, wenn die gemessene Konzentration über dem Schwellenwert liegt oder Zuordnen einer verringerten Wahrscheinlichkeit des Fortschreitens zu einer Notwendigkeit einer Nierenersatztherapie zu dem Subjekt, wenn die gemessene Konzentration unter dem Schwellenwert liegt,
(c) eine gemessene Urinkonzentration von HA und wobei der Korrelationsschritt das Vergleichen der gemessenen Konzentration mit einer Schwellenkonzentration umfasst und Zuordnen einer erhöhten Wahrscheinlichkeit des Fortschreitens zu einem schlechteren RIFLE-Stadium zu dem Subjekt, in Bezug auf das derzeitige RIFLE-Stadium des Subjektes, wenn die gemessene Konzentration über dem Schwellenwert liegt oder Zuordnen einer verringerten Wahrscheinlichkeit des Fortschreitens zu einem schlechteren RIFLE-Stadium zu dem Subjekt, in Bezug auf das derzeitige RIFLE-Stadium des Subjektes, wenn die gemessene Konzentration unter dem Schwellenwert liegt,

(d) eine gemessene Urinkonzentration von HA und wobei der Korrelationsschritt das Vergleichen der gemessenen Konzentration mit einer Schwellenkonzentration umfasst und Zuordnen einer erhöhten Wahrscheinlichkeit des Fortschreitens zu akutem Nierenversagen, wenn die gemessene Konzentration über dem Schwellenwert liegt oder Zuordnen einer verringerten Wahrscheinlichkeit des Fortschreitens zu akutem Nierenversagen zu dem Subjekt, wenn die gemessene Konzentration unter dem Schwellenwert liegt.

6. Ein Verfahren nach Anspruch 1, wobei das Subjekt zur Evaluation des Nierenstatus ausgewählt wird anhand

(a) des vorherigen Bestehens von einem oder mehreren bekannten Risikofaktoren für prärenales, immanent renales oder postrenales ARF bei dem Sujekt oder

(b) einer vorliegenden Diagnose von einem oder mehreren aus kongestiver Herzinsuffizienz, Präeklampsie, Eklampsie, Diabetes mellitus, Hypertonie, koronarer Herzkrankheit, Proteinurie, Niereninsuffizienz, glomerulärer Filtration unterhalb des normalen Bereichs, Zirrhose, Serumkreatinin oberhalb des normalen Bereichs, Sepsis, Verletzung der Nierenfunktion, verringerter Nierenfunktion oder ARF oder anhand eines laufenden oder vorangegangenen größeren gefäßchirurgischen Eingriffs, Koronararterien-Bypass-Operation oder einer anderen Herzoperation, oder anhand einer Exposition gegenüber NSAIDs, Cyclosporinen, Tacrolimus, Aminoglykosiden, Foscarnet, Ethylenglykol, Hämoglobin, Myoglobin, Ifosfamid, Schwermetallen, Methotrexat, röntgendichten Kontrastmitteln oder Streptozotocin.

7. Die Verwendung von HA zur Risikostratifizierung zum Identifizieren von Subjekten, die von einer zukünftigen Verletzung der Nierenfunktion, von zukünftigem Fortschreiten zu einer verringerten Nierenfunktion, von zukünftigem Fortschreiten zu ARF gefährdet sind und zur Prognose des Nierenstatus eines Subjektes, das keine Nierenersatztherapie erhält, oder eines Subjektes, das kein akutes Nierenversagen hat oder zum Zuordnen einer erhöhten Wahrscheinlichkeit des Fortschreitens zu einem schlechteren RIFLE-Stadium zu dem Subjekt, in Bezug auf das derzeitige RIFLE-Stadium des Subjektes, wobei das Subjekt (i) keinen 1,5-fachen oder höheren Anstieg von Serumkreatinin über einem Ausgangswert, der vor dem Zeitpunkt, an dem die Körperflüssigkeitsprobe erhalten wurde, bestimmt wurde, erfahren hat und/oder (iii) keinen Anstieg von Serumkreatinin von 0,3 mg/dl oder mehr über einen Ausgangswert, der vor dem Zeitpunkt, an dem die Körperflüssigkeitsprobe erhalten wurde, bestimmt wurde, erfahren hat, wobei die Verwendung eine Urin-Messung von HA umfasst.

## Revendications

1. Un procédé d'évaluation de l'état rénal chez un sujet qui ne reçoit pas une thérapie de remplacement rénal, consistant à :

réaliser un ou plusieurs dosages configurés pour détecter un ou plusieurs biomarqueurs, les biomarqueurs comprenant de l'acide hyaluronique (HA), sur un échantillon de fluide corporel obtenu chez le sujet pour fournir un ou plusieurs résultats de dosages ; et
corréler le ou les résultats du ou des dosages avec une ou plusieurs stratifications de risque pour identifier les sujets à risque pour une lésion future de la fonction rénale, pour une progression future vers un fonctionnement rénal diminué, pour une progression future vers une IRA et le pronostic de l'état rénal du sujet, dans lequel le sujet n'est pas en insuffisance rénale aiguë et dans lequel le sujet (i) n'a pas subi une augmentation de 1,5 fois ou plus de la créatinine sérique au-dessus d'une valeur de base déterminée avant le moment auquel l'échantillon de fluide corporel est obtenu, et/ou (iii) n'a pas subi une augmentation de 0,3 mg/dL ou plus de la créatinine sérique au-dessus d'une valeur de base déterminée avant le moment auquel l'échantillon de fluide corporel est obtenu.

2. Un procédé selon la revendication 1, dans lequel le sujet est au stade RIFLE 0.

3. Un procédé selon la revendication 2, dans lequel le sujet est au stade RIFLE 0, et ladite étape de corrélation consiste à attribuer une probabilité que le sujet atteindra le stade RIFLE R, I ou F en 72 heures, en 48 heures ou en 24 heures, dans lequel ledit ou lesdits résultats du ou des dosages comprennent éventuellement une concentration d'AH mesurée dans l'urine et ladite étape de corrélation consiste à comparer ladite concentration mesurée avec un seuil de concentration, et

attribuer une probabilité accrue de progresser vers le stade RIFLE R, I ou F chez le sujet, lorsque la concentration mesurée est au-dessus du seuil, ou attribuer une probabilité diminuée de progresser vers le stade RIFLE R, I ou F chez le sujet lorsque la concentration mesurée est en dessous du seuil.

4. Un procédé selon la revendication 2, dans lequel le sujet est au stade RIFLE 0, et ladite étape de corrélation consiste à attribuer une probabilité que le sujet atteindra le stade RIFLE I ou F en 72 heures, en 48 heures ou en 24 heures, dans lequel

(i) ledit ou lesdits résultats du ou des dosages comprennent éventuellement une concentration d'AH mesurée dans l'urine et ladite étape de corrélation consiste à comparer ladite concentration mesurée avec un seuil de concentration, et

attribuer une probabilité accrue de progresser vers le stade RIFLE I ou F chez le sujet, lorsque la concentration mesurée est au-dessus du seuil, ou attribuer une probabilité diminuée de progresser vers le stade RIFLE I ou F chez le sujet lorsque la concentration mesurée est en dessous du seuil ou

(ii) le sujet est éventuellement au stade RIFLE 0, et ladite étape de corrélation consiste à attribuer une probabilité que le sujet atteindra le stade RIFLE F en 72 heures, en 48 heures ou en 24 heures,

dans lequel ledit ou lesdits résultats du ou des dosages comprennent éventuellement une concentration d'AH mesurée dans l'urine et ladite étape de corrélation consiste à comparer ladite concentration mesurée avec un seuil de concentration, et attribuer une probabilité accrue de progresser vers le stade RIFLE F chez le sujet, lorsque la concentration mesurée est au-dessus du seuil, ou attribuer une probabilité diminuée de progresser vers le stade RIFLE F chez le sujet lorsque la concentration mesurée est en dessous du seuil.

5. Un procédé selon la revendication 1, dans lequel ledit ou lesdits résultats du ou des dosages comprennent

(a) une concentration d'AH mesurée dans l'urine et ladite étape de corrélation consiste à comparer ladite concentration mesurée avec un seuil de concentration, et attribuer une probabilité accrue de progresser vers un stade RIFLE s'aggravant chez le sujet, par rapport au stade RIFLE courant du sujet, lorsque la concentration mesurée est au-dessus du seuil, ou attribuer une probabilité diminuée de progresser vers un stade RIFLE s'aggravant chez le sujet, par rapport au stade RIFLE courant du sujet, lorsque la concentration mesurée est en dessous du seuil,

(b) une concentration d'AH mesurée dans l'urine et ladite étape de corrélation consiste à comparer ladite concentration mesurée avec un seuil de concentration, et attribuer une probabilité accrue de progresser vers un besoin de thérapie de remplacement rénal chez le sujet, lorsque la concentration mesurée est au-dessus du seuil, ou attribuer une probabilité diminuée de progresser vers un besoin de thérapie de remplacement rénal lorsque la concentration mesurée est en dessous du seuil,

(c) une concentration d'AH mesurée dans l'urine et ladite étape de corrélation consiste à comparer ladite concentration mesurée avec un seuil de concentration, et attribuer une probabilité accrue de progresser vers un stade RIFLE s'aggravant chez le sujet, par rapport au stade RIFLE courant du sujet, lorsque la concentration mesurée est au-dessus du seuil, ou attribuer une probabilité diminuée de progresser vers un stade RIFLE s'aggravant chez le sujet, par rapport au stade RIFLE courant du sujet, lorsque la concentration mesurée est en dessous du seuil, ou

(d) une concentration d'AH mesurée dans l'urine et ladite étape de corrélation consiste à comparer ladite concentration mesurée avec un seuil de concentration, et attribuer une probabilité accrue de progresser vers une insuffisance rénale aiguë lorsque la concentration mesurée est au-dessus du seuil, ou attribuer une probabilité diminuée de progresser vers une insuffisance rénale aiguë chez le sujet lorsque la concentration mesurée est en dessous du seuil.

6. Un procédé selon la revendication 1, dans lequel le sujet est choisi pour l'évaluation de l'état rénal en fonction de

(a) la pré-existence chez le sujet d'un ou plusieurs facteurs de risque connus pour une IRA, pré-rénale, rénale intrinsèque ou post-rénale, ou

(b) un diagnostic existant d'un ou plusieurs parmi une insuffisance cardiaque congestive, une pré-éclampsie, une éclampsie, le diabète sucré, l'hypertension, une maladie coronarienne, la protéinurie, une insuffisance rénale, une filtration glomérulaire en dessous de la plage normale, une cirrhose, une créatinine sérique supérieure à la plage normale, une sepsie, une lésion de la fonction rénale, une fonction rénale amoindrie, ou une IRA, ou sur la base du fait de subir ou d'avoir subi une chirurgie vasculaire majeure, un pontage aorto-coronarien, ou une autre chirurgie cardiaque, ou sur la base d'une exposition à des anti-inflammatoires non stéroïdiens, les cyclosporines, le tacrolimus, les aminoglycosides, le foscarnet, l'éthylène glycol, l'hémoglobine, la myoglobine, l'ifosfamide, des métaux lourds, du méthotrexate, des agents de contraste radio-opaques, ou de la streptozotocine.

7. L'utilisation de l'acide hyaluronique pour la stratification de risque pour identifier les sujets à risque pour une future lésion de la fonction rénale, pour la future progression vers une fonction rénale diminuée, pour la future progression vers une IRA et le pronostic de l'état rénal d'un sujet ne recevant pas une thérapie de remplacement rénal ou un sujet qui n'est pas en insuffisance rénale aiguë, ou pour attribuer une probabilité accrue de progresser vers un stade RIFLE s'aggravant chez un sujet, par rapport au stade RIFLE courant du sujet,
dans lequel le sujet (i) n'a pas subi une augmentation de 1,5 fois ou plus de la créatinine sérique au-dessus d'une valeur de base déterminée avant le moment auquel l'échantillon de fluide corporel est obtenu, et/ou (iii) n'a pas subi une augmentation de 0,3 mg/dL ou plus de la créatinine sérique au-dessus d'une valeur de base déterminée avant le moment auquel l'échantillon de fluide corporel est obtenu,
l'utilisation comprenant une mesure d'AH dans l'urine.

FIGURE 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 61386421 A
- US 2010081148 A
- US 6143576 A
- US 6113855 A
- US 6019944 A
- US 5985579 A
- US 5947124 A
- US 5939272 A
- US 5922615 A
- US 5885527 A
- US 5851776 A
- US 5824799 A
- US 5679526 A
- US 5525524 A
- US 5480792 A
- US 5631171 A
- US 5955377 A
- US 5571698 A, Ladner
- US 6057098 A

### Non-patent literature cited in the description

- Harrison's Principles of Internal Medicine. McGraw Hill, 1741-1830
- Current Medical Diagnosis & Treatment. McGraw Hill, 2008, 785-815
- Merck Manual
- **PRAUGHT ; SHLIPAK.** Curr Opin Nephrol Hypertens. 2005, vol. 14, 265-270
- **CHERTOW et al.** *J Am Soc Nephrol,* 2005, vol. 16, 3365-3370
- **LASSNIGG.** *J Am Soc Nephrol,* 2004, vol. 15, 1597-1605
- **BELLOMO et al.** *Crit Care,* 2004, vol. 8 (4), 204-12
- **KELLUM.** *Crit. Care Med.,* 2008, vol. 36, 141-45
- **RICCI et al.** *Kidney Int.,* 2008, vol. 73, 538-546
- **MEHTA et al.** *Crit. Care,* 2007, vol. 11, R31
- **MCCOLLOUGH et al.** *Rev Cardiovasc Med.,* 2006, vol. 7 (4), 177-197
- **RICHARD Y LIN et al.** Urinary hyaluronic acid is a Wilms' tumor marker. *Journal of pediatric surgery,* 1995, vol. 30 (2), 304-308
- **MARINO ASSELMAN et al.** Hyaluronan and Stone Disease. *AIP Conference Proceedings,* 2008, vol. 1049, 133-144
- **SHEN WENQING et al.** Changes and clinical significance of hyaluronic acid, procollagen type III, collagen type IV and laminin in the serum and urine in patients with chronic renal diseases. *Linchuang Yixue,* 2010, vol. 30 (6), 4-7
- **ZHANG et al.** Clinical significance of determination of changes of serum hyaluronic acid (HA) and plasma vascular endothelial growth factor (VEGF) contents alter treatment in patients with chronic nephropathy. *FANGSHE MIANYIXUE ZAZHI,* 2006, vol. 19 (4), 286-28
- Serum hyaluronic acid and procollagen III amino terminal propeptide in chronic renal failure. **HONKANEN et al.** American Journal Of Nephrology. S. Karger AG, 1991, vol. 11, 201-206
- Circulating hyaluronate. A potential marker of altered metabolism of the connective tissue in uremia. **HAELLGREN et al.** Nephron Experimental Nephrology. S. Karger AG, 1987, vol. 46, 150-154
- Fundamental Immunology. Raven Press, 1993
- **WILSON.** *J. Immunol. Methods,* 1994, vol. 175, 267-273
- **YARMUSH.** *J. Biochem. Biophys. Methods,* 1992, vol. 25, 85-97
- **WARD et al.** *Nature,* 1989, vol. 341, 544-546
- **VAN ERP et al.** *J. Immunoassay,* 1991, vol. 12, 425-43
- **NELSON ; GRISWOLD.** *Comput. Methods Program Biomed.,* 1988, vol. 27, 65-8
- **CWIRLA et al.** *Proc. Natl. Acad. Sci. USA,* 1990, vol. 87, 6378-82
- **DEVLIN et al.** *Science,* 1990, vol. 249, 404-6
- **SCOTT ; SMITH.** *Science,* 1990, vol. 249, 386-88
- **FISCHER et al.** *Intensive Care Med.,* 2003, vol. 29, 1043-51
- **BAGSHAW et al.** *Nephrol. Dial. Transplant.,* 2008, vol. 23, 1203-1210

- Merck Manual of Diagnosis and Therapy. Merck Research Laboratories, 1999
- **HANLEY, J. A. ; MCNEIL, B.J.** The meaning and use of the area under a receiver operating characteristic (ROC) curve. *Radiology,* 1982, vol. 143, 29-36
- **UCHINO S ; KELLUM JA ; BELLOMO R ; DOIG GS ; MORIMATSU H ; MORGERA S ; SCHETZ M ; TAN I ; BOUMAN C ; MACEDO E.** Ronco C: Acute renal failure in critically ill patients: a multinational, multicenter study. *Jama,* 2005, vol. 294, 813-818
- **MANNS B ; DOIG CJ ; LEE H ; DEAN S ; TONELLI M ; JOHNSON D ; DONALDSON C.** Cost of acute renal failure requiring dialysis in the intensive care unit: clinical and resource implications of renal recovery. *Crit Care Med,* 2003, vol. 31, 449-455
- **HANSELL P ; GORANSSON V ; ODLIND C ; GERDIN B ; HALLGREN R.** Hyaluronan content in the kidney in different states of body hydration. *Kidney Int,* 2000, vol. 58, 2061-2068
- **SIBALIC V ; FAN X ; LOFFING J ; WUTHRICH RP.** Upregulated renal tubular CD44, hyaluronan, and osteopontin in kdkd mice with interstitial nephritis. *Nephrol Dial Transplant,* 1997, vol. 12, 1344-1353
- **LEWINGTON AJ ; PADANILAM BJ ; MARTIN DR ; HAMMERMAN MR.** Expression of CD44 in kidney after acute ischemic injury in rats. *Am J Physiol Regul Integr Comp Physiol,* 2000, vol. 278, R247-254
- **SANO N ; KITAZAWA K ; SUGISAKI T.** Localization and roles of CD44, hyaluronic acid and osteopontin in IgA nephropathy. *Nephron,* 2001, vol. 89, 416-421
- **MELIN J ; HELLBERG O ; FUNA K ; HALLGREN R ; LARSSON E ; FELLSTROM BC.** Ischemia-induced renal expression of hyaluronan and CD44 in diabetic rats. *Nephron Exp Nephrol,* 2006, vol. 103, e86-94
- **YANG J ; LIU Y.** Dissection of key events in tubular epithelial to myofibroblast transition and its implications in renal interstitial fibrosis. *Am J Pathol,* 2001, vol. 159, 1465-1475
- **OKAJIMA K.** Regulation of inflammatory responses by natural anticoagulants. *Immunol Rev,* 2001, vol. 184, 258-274
- **WANG X ; HUANG G ; MEI S ; QIAN J ; JI J ; ZHANG J.** Over-expression of C/EBP-alpha induces apoptosis in cultured rat hepatic stellate cells depending on p53 and peroxisome proliferator-activated receptor-gamma. *Biochem Biophys Res Commun,* 2009, vol. 380, 286-291
- **TAKEDA K ; KOJIMA Y ; IKEJIMA K ; HARADA K ; YAMASHINA S ; OKUMURA K ; AOYAMA T ; FRESE S ; IKEDA H ; HAYNES NM.** Death receptor 5 mediated-apoptosis contributes to cholestatic liver disease. *Proc Natl Acad Sci U S A,* 2008, vol. 105, 10895-10900
- **WOLF G.** Renal injury due to renin-angiotensin-aldosterone system activation of the transforming growth factor-beta pathway. *Kidney Int,* 2006, vol. 70, 1914-1919
- **BASILE DP.** The endothelial cell in ischemic acute kidney injury: implications for acute and chronic function. *Kidney Int,* 2007, vol. 72, 151-156
- **HVIDBERG V ; JACOBSEN C ; STRONG RK ; COWLAND JB ; MOESTRUP SK ; BORREGAARD N.** The endocytic receptor megalin binds the iron transporting neutrophil-gelatinase-associated lipocalin with high affinity and mediates its cellular uptake. *FEBS Lett,* 2005, vol. 579, 773-777
- **MORI K ; NAKAO K.** Neutrophil gelatinase-associated lipocalin as the real-time indicator of active kidney damage. *Kidney Int,* 2007, vol. 71, 967-970
- **MORI K ; LEE HT ; RAPOPORT D ; DREXLER IR ; FOSTER K ; YANG J ; SCHMIDT-OTT KM ; CHEN X ; LI JY ; WEISS S.** Endocytic delivery of lipocalin-siderophore-iron complex rescues the kidney from ischemia-reperfusion injury. *J Clin Invest,* 2005, vol. 115, 610-621
- **NICKOLAS TL ; O'ROURKE MJ ; YANG J ; SISE ME ; CANETTA PA ; BARASCH N ; BUCHEN C ; KHAN F ; MORI K ; GIGLIO J.** Sensitivity and specificity of a single emergency department measurement of urinary neutrophil gelatinase-associated lipocalin for diagnosing acute kidney injury. *Ann Intern Med,* 2008, vol. 148, 810-819
- **PALEVSKY PM ; ZHANG JH ; O'CONNOR TZ ; CHERTOW GM ; CROWLEY ST ; CHOUDHURY D ; FINKEL K ; KELLUM JA ; PAGANINI E ; SCHEIN RM.** Intensity of renal support in critically ill patients with acute kidney injury. *N Engl J Med,* 2008, vol. 359, 7-20
- **BONE RC ; BALK RA ; CERRA FB ; DELLINGER RP ; FEIN AM ; KNAUS WA ; SCHEIN RM ; SIBBALD WJ.** Definitions for sepsis and organ failure and guidelines for the use of innovative therapies in sepsis. The ACCP/SCCM Consensus Conference Committee. *American College of Chest Physicians/Society of Critical Care Medicine,* 1992, vol. 136, e28
- **BELLOMO R ; RONCO C ; KELLUM JA ; MEHTA RL ; PALEVSKY P.** Acute renal failure - definition, outcome measures, animal models, fluid therapy and information technology needs: the Second International Consensus Conference of the Acute Dialysis Quality Initiative (ADQI) Group. *Crit Care,* 2004, vol. 8, 204-212

• **RONCO P ; LELONGT B ; PIEDAGNEL R ; CHATZ-IANTONIOU C.** Matrix metalloproteinases in kidney disease progression and repair: a case of flipping the coin. *Semin Nephrol,* 2007, vol. 27, 352-362